# EUROPEAN PATENT SPECIFICATION

(11) **EP 2 196 532 B1**
(45) Date of publication and mention of the grant of the patent: **10.12.2014**
(21) Application number: 08805322.8
(22) Date of filing: 11.07.2008
(51) Int. Cl.: C12N 1/10, A61K 39/002, A61K 35/68, G01N 33/53, C12R 1/90

(54) **USE OF A NEW ISOLATE OF NEOSPORA CANINUM FOR THE DEVELOPMENT OF DIAGNOSTIC TESTS AND FOR PREPARATION OF PRODUCTS FOR TREATMENT AND PREVENTION OF THE INFECTION CAUSED BY NEOSPORA**
VERWENDUNG EINES NEUES ISOLATS AUS NEOSPORA CANINUM ZUR ENTWICKLUNG DIAGNOSTISCHER TESTS UND ZUR HERSTELLUNG VON PRODUKTEN ZUR BEHANDLUNG UND PRÄVENTION VON NEOSPORA-INFEKTIONEN
UTILISATION D'UN NOUVEL ISOLAT DE NEOSPORA CANINUM POUR LE DEVELOPPEMENT DE TESTS DE DIAGNOSTIC ET POUR LA FABRICATION DE PRODUITS UTILISES DANS LE TRAITEMENT ET LA PREVENTION D'UNE INFECTION CAUSEE PAR NEOSPORA

(30) Priority: 13.07.2007 ES 200701978
(43) Date of publication of application: 16.06.2010
(73) Proprietor: Universidad Complutense de Madrid, 28040 Madrid (ES)
(72) Inventor: ORTEGA MORA, Luis, E-28040 Madrid (ES); REGIDOR CERRILLO, Javier, E-28040 Madrid (ES); GÓMEZ BAUTISTA, Mercedes, E-28040 Madrid (ES); AGUADO MARTÍNEZ, Adriana, E-28040 Madrid (ES); COLLANTES FERNÁNDEZ, Esther, E-28040 Madrid (ES); ADURIZ, Gorka, E-28040 Madrid (ES)
(74) Representative: Carpintero Lopez, Francisco
(86) International application number: PCT/ES2008/000492
(87) International publication number: WO 2009/010614

(56) References cited:
- EP-A1- 1 655 378
- EP-A2- 1 710 309
- WO-A1-02/10340
- WO-A1-95/25541
- WO-A1-99/20303
- WO-A1-2007/073914
- FR-A1- 2 771 101
- PEREIRA GARCIA-MELO D ET AL: "Pathogenic characterization in mice of Neospora caninum isolates obtained from asymptomatic calves", PARASITOLOGY, vol. 137, no. 7, June 2010 (2010-06), pages 1057-1068, XP009143159, ISSN: 0031-1820

### SEQUENCE LISTING

**<110> Universidad Complutense de Madrid**
<120> Use of a new isolate of *Neospora caninum* for the development of diagnostic tests and for manufacture of products for treatment and prevention of the infection caused by *Neospora*.
<160> 42
<210> 1
   <211> 313
   <212> DNA
   <213> *Neospora caninum*
<400> 1
   gacgcctttt caactgtcaa cagatacttt tttctagttc gactcaccgc aacacatgcc 60
   cacagcgaca gatacgcgcc atatgcaggc gcgggcaggt gcatgcacgc ttgcgacaag 120
   tatatatata aatatatata tgtatatatg tataaatata tatatatgta tatatgtata 180
   aatatatata tatatatgta tatatgtata aatatatata tatatgtata tgtatatata 240
   tgcaaggccg gtgtgtggac cttcggctga agctgaggag ttcgtcgagg tgtatggcga 300
   gtgattggaa gtt 313
<210> 2
   <211> 253
   <212> DNA
   <213> *Neospora caninum*
<400> 2
   gaaagacgat tccccatttc aaactgcaaa cgtctcagca aatacaagtg acctcttgcg  60
   cccggagttc ttcgtatgca cacacatcta catatatata tatatataca tatatgcatt 120
   tccatatgga tttacatgtg tagaaaatat agatgtgtat gtttgatttt gtgactctcg 180
   atgcatctgc ctgcacgcgc atgtggaggt tttcttgtga atgcagcagg cgagaggaaa 240
   gggagtttgt ggg                                                    253
<210> 3
   <211> 142
   <212> DNA
   <213> *Neospora caninum*
<400> 3
<210> 4
   <211> 198
   <212> DNA
   <213> *Neospora caninum*
<400> 4
   ctgtcgtatg aatgcatata tatatatata tatatatata tataattatg tagatgtacc  60
   tgtatctata catatgtacg tgtgccttga ttgctgcacc atgccgtttt gtggcctgtt 120
   ttacgaagga gaagggattc ccctttcatt tcatttgcag aaaattgaca agagctgcag 180
   cctcaccatg ccatgtgg                                               198
<210> 5
   <211> 235
   <212> DNA
   <213> *Neospora caninum*
<400> 5
<210> 6
   <211> 267
   <212> DNA
   <213> *Neospora caninum*
<400> 6
<210> 7
   <211> 403
   <212> DNA
   <213> *Neospora caninum*
<400> 7
   cagagctcgc caatagatct gaggaaccct caaagtgcaa agtgcatgtt tgtatccctt  60
   gaagctatat atatatatat atatatatat aacttcatat gcatatgaat gtctggtctc 120
   gcactcatgg gattttgtag gccgatgcgt gcttacccat cgacccctct gcatagctgt 180
   ccatctgtgt gtctgcataa tatgcacaca tgcatctaaa gctgcgtgca tgtaactatt 240
   attcgtgcat gcgcaaaata ctgacatcca tatatatata tatatatata tgtacatgca 300
   taccacgtgt gtacgattac gttagcagag gcggccgatt gcgcgaccac gacagagggg 360
   ttggaccgtg gtcatctttg ttactagcga tcaggtgcat tga                   403
<210> 8
   <211> 254
   <212> DNA
   <213> *Neospora caninum*
<400> 8
   tccaactcgg cgagatacgc agagagggct gttcctggca acacgacagt gactctccat  60
   acatcccgcg ctgctcccgt cgctgcacac ttgcatgcac ctatacgtct tcagaccata 120
   atatatatat atatatatat aggggatagg cgacgacggc gagtgttgcg tgtgtgcgtt 180
   ccctgcggag agagcgctag ggagtcgatt gacaggggct gtgggggcct gcttacggga 240
   gaggagggac tgaa                                                   254
<210> 9
   <211> 193
   <212> DNA
   *<213> Neospora caninum*
<900> 9
<210> 10
   <211> 292
   <212> DNA
   *<213> Neospora caninum*
<400> 10
   tcttctcttc tctccgcttt ctccaaattt aaaaggcctt tcggaagtct cccgagctgt 60
   gacctccctc gtgtcgtact cgtagtagta gtactactac tactactact agaagaagaa 120
   gaagaagaag aagaagaaga agaagaagaa gaagaagaag aagaagaaga agaagaagat 180
   gatgatgatg atgatgatga tgatgatgac aatctacgtc aatctacgtc aaacagggat 240
   gtgcaagtac ctgcaaggaa gagctatagg aagagagaaa ggagaataaa gg 292
<210> 11
   <211> 242
   <212> DNA
   <213> *Neospora caninum*
<400> 11
   tgtttttttg agttccttgc aaaactcggg agaaattcgg gacccaaggg gaagtggaca 60
   ctcggggggc tgagggcaac cgtctcgttt ctggccagga gcaaaagcta ctcaagagac 120
   agcaagatcg gcgcatttcc gctccgatgc tctgcgtgtg tgtgtgtgtg tgtgtgtgtg 180
   tgtgtgtgcg cccgagtctc gtttgttatt aattgccgtc gtctgcgtgt tcggagtctc 240
   tt 242
<210> 12
   <211> 289
   <212> DNA
   <213> *Neospora caninum*
<400> 12
   cggtggttct tcagcagcgt tccaaaaaga accagtgaat aggtcggagc ccggtactcc 60
   tggatggtga gacggttgaa tcgggctggt ccctaatcgt ttaccacgag ttccctccaa 120
   catgcatata tacatgtaca tacatacata cctacataca tacatacata catttccccc 180
   cgctctttct caaacgtttc ctttggccga gcttgcacat atacaaagta taacatccag 240
   gcggcgatgg aatcagcatg ttcacttttg gtgaagggga gggtagaaa             289
<210> 13
   <211> 18
   <212> DNA
   <213> *Neospora caninum*
<400> 13
   tcaacctttg aatcccaa 18
<210> 14
   <211> 18
   <212> DNA
   <213> Neospora *caninum*
<400> 14
   cgagccaaga catccatt 18
<210> 15
   <211> 19
   <212> DNA
   <213> *Neospora caninum*
<400> 15
   tactactccc tgtgagttg 19
<210> 16
   <211> 18
   <212> DNA
   <213> *Neospora caninum*
<400> 16
   tctcttccct caaacgct 18
<210> 17
   <211> 20
   <212> DNA
   <213> *Neospora* caninum
<400> 17
   acgacgcctt ttcaactgtc 20
<210> 18
   <211> 20
   <212> DNA
   <213> *Neospora* caninum
<400> 18
   cgtccgttct cctctctcag 20
<210> 19
   <211> 20
   <212> DNA
   <213> *Neospora caninum*
<400> 19
   ggagaacgga cgacgttaag 20
<210> 20
   <211> 20
   <212> DNA
   <213> Neospora caninum
<400> 20
   cccacaaact ccctttcctc 20
<210> 21
   <211> 20
   <212> DNA
   <213> *Neospora caninum*
<400> 21
   ctcttctcac cagcccagtc 20
<210> 22
   <211> 20
   <212> DNA
   <213> *Neospora caninum*
<400> 22
   gctgttacaa cccacggaac 20
<210> 23
   <211> 21
   <212> DNA
   <213> *Neospora caninum*
<400> 23
   gactttggtt tgccactgtc g 21
<210> 24
   <211> 20
   <212> DNA
   <213> *Neospora caninum*
<400> 24
   tccgacatct acggacatcg 20
<210> 25
   <211> 20
   <212> DNA
   <213> *Neospora caninum*
<400> 25
   agaagaagaa acgcggaatg 20
<210> 26
   <211> 20
   <212> DNA
   <213> *Neospora caninum*
<400> 26
   tctgaaacga attcccttgg 20
<210> 27
   <211> 20
   <212> DNA
   <213> *Neospora caninum*
<400> 27
   aatcaggact ccgtcacacc 20
<210> 28
   <211> 19
   <212> DNA
   <213> *Neospora caninum*
<400> 28
   ccagagagtc ccacatctc 19
<210> 29
   <211> 18
   <212> DNA
   <213> *Neospora caninum*
<400> 29
   tccgcgtgtg gtttatct 18
<210> 30
   <211> 20
   <212> DNA
   <213> *Neospora caninum*
<400> 30
   tgcgcatgca cgaataatag 20
<210> 31
   <211> 21
   <212> DNA
   <213> *Neospora caninum*
<400> 31
   gcactcatgg gattttgtag g 21
<210> 32
   <211> 21
   <212> DNA
   <213> *Neospora caninum*
<400> 32
   aaaaatcaat gcacctgatc g 21
<210> 33
   <211> 20
   <212> DNA
   <213> *Neospora caninum*
<400> 33
   gatccaactc ggegagatac 20
<210> 34
   <211> 20
   <212> DNA
   <213> *Neospora caninum*
<400> 34
   cgttcccttc ccaaatcttc 20
<210> 35
   <211> 20
   <212> DNA
   <213> *Neospora caninum*
<400> 35 20
   acactcgcct ttcctttgtg 20
<210> 36
   <211> 20
   <212> DNA
   <213> *Neospora caninum*
<400> 36
   cacacaggcc agttgaaaag 20
<210> 37
   <211> 21
   <212> DNA
   <213> *Neospora caninum*
<400> 37
   ctatcacagc cgtgagtgtt g 21
<210> 38
   <211> 18
   <212> DNA
   <213> *Neospora caninum*
<400> 38
   cgcgctatcc tttattct 18
<210> 39
   <211> 18
   <212> DNA
   <213> *Neospora caninum*
<400> 39 18
   ccagcatttc ttctcctt 18
<210> 40
   <211> 20
   <212> DNA
   <213> *Neospora caninum*
<400> 40 20
   caaaaacgca ctttctttcg 20
<210> 41
   <211> 20
   <212> DNA
   <213> *Neospora caninum*
<400> 41 20
   tgtgagcata acggtggttc 20
<210> 42
   <211> 20
   <212> DNA
   <213> *Neospora caninum*
<400> 42
   tttctaccct ccccttcacc 20

## Description

### Technical Field. Object of the invention.

The present invention is comprised within the field of animal health and relates, as expressed in the title of this specification, to the development of diagnostic tests for the detection of *Neospora caninum* in animals and to the manufacture of pharmaceutical compositions for the prevention and the treatment of neosporosis therein.

More specifically, the invention relates to the use of the isolate Nc-Spain 7 of *N. caninum* the relevant feature of which is the ease of conversion between the tachyzoite and bradyzoite stages under *in vitro* conditions, and for that reason it is a suitable candidate for the manufacture of new vaccine and therapeutic products against neosporosis, and for the development of new diagnostic methods to detect the infection caused by the *Neospora caninum* parasite in animals from biological samples.

### Prior State of the Art

*Neospora caninum* is a parasite belonging to the phylum *Apicomplexa* in which the genera *Plasmodium, Babesia, Cryptosporidium, Eimeria* and *Toxoplasma* are grouped, which include some of the most important disease-causing organisms known to man. *N. caninum* was first described in 1984 as a protozoan able to cause encephalitis and myositis in dogs, and shortly thereafter it was recognized as an abortion- and neonatal mortality-causing agent in cattle. Infections caused by the parasite in other hosts, including several hoofed animals and canines, have also been described in recent years, however the importance of neosporosis in cattle and in dogs, which acts as a definitive host in the biological cycle, stands out (Dubey et al., 2006, J. Comp. Path. 134: 267-289).

Until now three stages in the biological cycle of *N. caninum* have been described: sporozoites, which are developed in the oocysts eliminated in the feces of the definitive host and are able to infect the intermediate host; tachyzoites, the fast multiplication form, responsible for the acute phase of the infection and its propagation to other tissues; and bradyzoites, the slow multiplication stage of the parasite giving rise to tissue cysts in which the parasite remains stationed during the chronic phase until its reactivation.

Bovine neosporosis is considered a parasitic disease with cosmopolitan distribution and one of the most frequent causes of reproductive failure in cattle in several producing countries in which it has been studied, including Spain (Pereira-Bueno et al., 2003, Vet Parasitol 111: 143-152). The most important clinical manifestation of the infection is abortion, which generally takes place between the fifth and seventh month of gestation. Further, live infected calves can be born that may have neuromuscular problems up to two months post-partum. However, the most frequent manifestation is the birth of clinically healthy but chronically infected calves. With respect to the transmission of the disease, the fundamental route is endogenous transplacental infection, although the involvement of horizontal transmission has also been demonstrated (Trees & Williams, 2005, Trends Parasitol. 21 (12): 558-561, Dubey et al., 2006, J. Comp. Path. 134: 267-289).

The diagnostic methods used to detect infection by *N. caninum,* generally in adult bovines, mainly consist of techniques based on the detection of serum antibodies specific for the parasite. To that end, different immunoassays have been designed comprising indirect immunofluorescence assay (IFA), several immunoenzymatic assays (ELISA) and immunoblot (IB) (Ortega-Mora et al., 2006, Acta Parasitológica 51: 1-14). The antigens used in these serologic tests are from tachyzoites obtained in cell cultures of bovine and canine isolates of *N. caninum.* Whole tachyzoites, cell extracts obtained therefrom, antigenic proteins purified by affinity from these cell extracts or recombinant proteins, as described in patent application WO97/39009, have been used as antigens in the development of these techniques. However, it should be explained that even though a positive serologic result aids in identifying an infected adult animal, a negative result does not definitively discard infection. Different studies have demonstrated that serum antibodies for *N. caninum* can fluctuate depending on the age and the state of gestation, and seroconvert into titers which are normally considered negative (Pereira-Bueno et al., 2000, in: Hemphill A & Gottstein B (Eds), Int J Parasitol 30: 906-909; Quintanilla-Gozalo et al., 2000, in: Hemphill A, Gottstein B (Eds), Int J Parasitol 30: 900-906; Maley et al., 2001, Vet Parasitol. 5; 96 (1): 1-9). The low levels of antibodies for *N. caninum* have mainly been associated to chronic infections, in which there is a predominance of the bradyzoite stage. It has therefore been suggested that a serologic test using bradyzoite-derived antigens could be more suitable for the identification of animals persistently infected (Maley et al., 2001, Vet Parasitol. 5; 96(1):1-9).

On the other hand, studies conducted for the development of vaccines for the protection against neosporosis have included the evaluation of inactivated vaccines, attenuated vaccines, vaccines developed from recombinant antigenic proteins and DNA vaccines with variable results. One of the few studies conducted with bovines demonstrated that the vaccine developed from dead tachyzoites emulsified with the adjuvant POLYGEN™, described in patent application WO99/20303, was able to originate a slight immune cell response (Andrianarivo et al., 1999, Int. J. Parasitol. 29, 1613-1625), although in gestating reproducers was unable to protect against fetal infection (Andrianarivo et al., 2000, Int J Parasitol. 30(9): 985-90). It is currently the only vaccine against *N. caninum* marketed and registered in the United States called NeoGuard™ the efficacy of which has recently been evaluated in the field (Romero et al., 2004, Vet Parasitol. 123(3-4): 149-59).

It is necessary to stress that all attenuated live vaccines, as described in patents EP0841392 and WO2004/026903, and inactivated vaccines, such as those described in patents EP0898969 and WO99/20303, have been prepared from tachyzoites maintained in cell culture or from recombinant antigenic proteins which are expressed in this stage of the biological cycle, as described in patent application EP0953641, and not from the bradyzoite stage, predominant in chronic infections.

Nowadays, the processes involved in tachyzoite-bradyzoite conversion in *N. caninum* are not known in detail. In recent years, they were obtained from purified tissue cysts from experimentally infected animals in a single study (McGuire et al., 1997, J. Parasitol. 83: 647-651), although the elevated number of bradyzoites necessary for the different studies has brought up the development of new methods for obtaining them *in vitro.* These methods have mainly been based on the use of chemical compounds such as sodium nitroprussiate as an inducer of the conversion into different cell lines (Vonlaufen et al., 2002, Int. J. Parasitol. 32: 1253-1265; Risco-Castillo et al., 2004, J. Parasitol. 90(3): 466-70). And it should be stressed that in studies conducted with different isolates (Nc-Liverpool, Nc-1, Nc-2 and Nc- SweB1) significant differences were also observed in the percentage of conversion between both stages depending on the isolate used (Weiss et al., 1999, Int. J. Parasitol. 29: 1713-1723; Vonlaufen, 2002, Int. J. Parasitol. 32: 1253-1265). These differences could be attributable to the biological diversity existing between different isolates of *N. caninum.*

Comparative studies of the biological properties of different isolates of *N. caninum* have demonstrated the existence of biological variability, including differences both in their pathogenicity and in their genetic profiles (Atkinson et al., 1999, Parasitology 118 (Pt 4): 363-370; Schock et al., 2001, Parasitology 123 (Pt 1): 13-23, Regidor-Cerrillo et al., 2006, J. Parasitol, 92(3): 517-524). In fact, patent application WO02/103430 describes live vaccines obtained from an isolate naturally having a low pathogenicity in an experimental murine model.

### Detailed Description of the Invention

The inventors have obtained and characterized a new isolate of *Neospora caninum* with a high *in vitro* capability of conversion between the tachyzoite and bradyzoite stages of its biological cycle and applicable in the development of diagnostic tests and products for the treatment and prevention of the infection caused by this parasite.

The new isolate is called Nc-Spain 7 in compliance with the international code of nomenclature of microorganisms, and it has been identified and characterized by means of different *in vitro* studies and experimental *in vivo* infections as a new isolate of the species *Neospora caninum.* A sample of the culture of tachyzoites of the isolate Nc-Spain 7 was deposited on 20/09/2005, according to the provisions of the Budapest Treaty on the International Recognition of the Deposit of Microorganisms for the Purposes of Patent Procedure, in the Culture Collection of Algae and Protozoa (CCAP) located in the Dunstaffnage Marine Laboratory, Dunbeg, OBAN, Argyll PA37 1QA, Scotland, Great Britain, with access number CCAP 2051/1.

In this description, the expression isolated parasite or simply isolate of the invention refers to the new isolate of *Neospora caninum*, Nc-Spain 7, obtained in any stage of its biological cycle. In the same manner, the expression extracts, cell lysates, polypeptides and polynucleotides obtained from the isolate of the invention relates to extracts, cell lysates, polypeptides and polynucleotides obtained from the isolate of the invention in any stage of its biological cycle.

Said isolate was obtained from the nervous tissue of a congenitally infected calf of the species *Bos taurus* of the Holstein Friesian race as described in Example 1.

To that end, before performing the isolation, the presence of the parasite in samples of nervous tissue from the infected calf was confirmed by means of nested PCR developed for the specific detection of the DNA of the parasite in clinical samples. The specific amplification of the ITS-1 region allowed detecting the presence of DNA of this parasite in said samples. Furthermore, the histological and immunohistochemical analysis of the same nervous tissue once again confirmed the presence of the parasite in this tissue, with the observation of lesions compatible with the infection by *N. caninum,* as well as the detection of cysts by means of the use of a rabbit polyclonal antibody obtained for tachyzoites of *N. caninum.* Immediately after, it was isolated using as a first step athymic mice of the BALB/c strain sensitive to the infection which were inoculated with different amounts of the nervous tissue. The onset of clinical signs compatible with the neosporosis allowed, in a second step, its *in vitro* isolation in cell cultures of the cell line MARC-145 (African vervet monkey (*Cercopithecus aethiops)* kidney cells).

Its genetic characterization was performed by means of the study of microsatellite sequences, which turns out to be a technique suitable for the identification and discrimination of different isolates of *N. caninum,* as described in Example 2 of this specification. With respect to its pathogenic characterization, it was performed in an experimental murine model, as described in Example 3 of this patent application. In said model, some of the mice inoculated with tachyzoites of the isolate Nc-Spain 7 developed clinical signs compatible with the infection by *N. caninum* and the presence of DNA of the parasite was detected in blood, lung and brain by means of nested PCR of the previously mentioned ITS-1 region. On the other hand, the capability of the isolate Nc-Spain 7 of inducing an immune response specific for *N. caninum* in the mice infected according to the experimental murine model was verified in the immunogenicity assays described in Example 4 by means of an ELISA technique for the detection of the immunoglobulins IgG2a and IgG1 specific for *Neospora*.

The capability of *in vitro* conversion of the isolate Nc-Spain 7 between the tachyzoite and bradyzoite stages was also assessed by means of a study in cell cultures of line MARC-145 and using sodium nitroprussiate as a stressing agent. The conversion between both stages was evaluated by means of the detection of parasitophorous vacuoles by the indirect immunofluorescence technique with parasites expressing the protein BAG1, expressed only in the bradyzoite stage, against the parasitophorous vacuoles with parasites expressing the protein NcSAGI, specific of the tachyzoite stage as described in Example 5.

The obtained results accordingly demonstrated that the isolate Nc-Spain 7 belongs to the species *N. caninum* and has an elevated capability of *in vitro* conversion from the tachyzoite stage to the bradyzoite stage, which makes it very useful for the development of new diagnostic tests, as well as for the development of new pharmaceutical compositions for the treatment and prevention of infection by *Neospora,* as described in Examples 7 and 8.

### Cell cultures

Another aspect of the invention relates to biologically pure cell cultures infected by the isolate Nc-Spain 7 of the invention which is maintained *in vitro* by means of successive passages in cultures of different cell lines.

Tachyzoites and bradyzoites of the isolate of the invention are obtained, by means of these cell cultures, which are used in obtaining polypeptides, antigens, antibodies, nucleic acids and other compounds of use in the preparation of products for the diagnosis, treatment and prevention of neosporosis.

The expression "biologically pure cell culture" of the isolate of the invention relates to a culture of the isolate Nc-Spain 7 maintained in a continuous manner substantially free of other organisms, except the host cells in which the parasite multiplies. It is considered that a culture is substantially free of other organisms if after the normalized processes for the recovery of the isolate they result in a preparation with at least 95%, and preferably 99% or more, of the parasite.

The preferred cell culture is of vervet monkey kidney cells among which lines MA-1 04, MARC-145, and Vero cells, can be mentioned.

### Antibodies

The disclosure also comprises the antibodies which react specifically against the antigens from the isolate of the invention.

The term "antibody" relates to an immunoglobulin molecule produced by an animal organism able to bind to a specific epitope of an antigen. The antibodies can be formed by a polyclonal mixture or can be monoclonal antibodies. They can also be formed by intact natural or recombinant immunoglobulins or fragments of these antibodies which retain their capability of recognition and binding of the target epitope and include the Fc fragment and the antigen binding fragments, F(ab) and F(ab)₂, as well as single chains (heavy chain (H) and light chain (L)) fused or not to other molecules.

The antigen from the isolate Nc-Spain 7, against which the antibody specifically reacts, is preferably selected from the group consisting of the isolate itself in any of its stages of the biological cycle, extracts or cell lysates, antigenic polypeptides, or mixtures thereof.

More preferably, the antigen is selected from the group consisting of tachyzoites and/or bradyzoites, and antigenic polypeptides. The antigenic polypeptides are the obtained from the isolate itself or by means of recombinant technology from polynucleotides of the isolate of the invention.

To obtain polyclonal antibodies, different previously selected animals species (mice, rabbits, goats or horses, among others) are immunized with an immunogen formed by the isolate itself of the invention inactivated in any of the stages of its biological cycle, or one or several natural and/or recombinant proteins, purified or not, in formulation or not with an adjuvant. The serum of the immunized animal is subsequently collected and there is an option of treating it for the purpose of producing an enrichment of the immunoreactive antibodies of interest according to different conventional processes.

With respect to the monoclonal antibodies, they are defined as the antibodies produced by a clonal population of cells generated from a single immortalized cell and, therefore, are identical and able to specifically recognize a single epitope.

The monoclonal and polyclonal antibodies developed against certain epitopes are particularly useful for the diagnosis of the disease, and those having a neutralizing activity against the parasite are very useful in passive immunotherapies. Their applications include diagnostic tests by means of immunoassay such as, for example, competitive ELISA or agglutination, immunohistochemical tests, and other applications such as *in vitro* evaluation of the capability of adhesion/invasion of the parasite, the detection and prior selection of antigens for the development of new vaccines, purification of proteins of interest, and the search in genomic and complementary DNA gene libraries for the identification of genes of interest.

The antibodies of the present disclosure specifically bind to the isolate itself of the invention in any of its stages of the biological cycle, extracts and/or cell lysates, antigenic polypeptides, or mixtures thereof, and are suitable for detecting the presence of the *N. caninum* parasite or of the infection caused by the same in animals from biological samples.

The antibody of the disclosure is preferably monoclonal.

### Polypeptides

Polypeptides obtained from the isolated parasite are also part of the disclosure. In this description, the term "polypeptide" includes the polypeptides obtained from the isolate of the invention in any stage of its biological cycle, from extracts and/or cell lysates, or mixtures thereof. The recombinant polypeptides produced in heterologous expression systems after the transcription and translation of nucleotide sequences obtained from the isolate of the invention in any stage of its biological cycle are also included.

The polypeptide sequences of the disclosure include not only "identical" sequences, but also those which are "substantially identical" thereto. The polypeptides can vary by means of performing deletions, insertions and substitutions in their amino acid sequence, giving rise to different but substantially identical polypeptides. The term "identical or percentage of identity" of sequences for two or more polynucleotides and polypeptides relates to two or more sequences which are the same or have a specific percentage of nucleotide or amino acid residues which are equal, respectively, when these are aligned for maximum correspondence, compared and this percentage is calculated by means of the use of algorithms used in their comparison or by visual inspection. The term "substantially identical" relates to polynucleotide or polypeptide sequences having at least 60%, preferably 80% and much more preferably 90-95% identity in the amino acid or nucleotide residues, when they are compared, aligned for maximum correspondence and their percentage of identity calculated by the already mentioned methods. The BLAST program is an example of the programs used to calculate the percentage of identity and similarity between sequences (http://www.ncbi.nlm.nih.gov/blast).

The polypeptides of the disclosure have diagnostic or immunogenic interest. In the first case, they are part of immunoassay tests as an antigen for the detection of antibodies specific for *N. caninum,* and in the second case, they are part of pharmaceutical compositions for the prevention and treatment of infections caused by *Neospora* and/or obtaining antibodies.

One option for the isolation of antigenic polypeptides of the new isolate Nc-Spain 7 comprises the use of normalized techniques for purification of proteins or polypeptides.

An alternative process is based on using the polynucleotides obtained from the isolate of the invention for the expression of recombinant proteins by means of the introduction of the polynucleotides encoding the proteins of interest in heterologous expression systems suitable for their production, subsequently performing a purification process. These systems include prokaryotic cells (for example *Escherichia coli*), yeasts (for example *Saccharomyces cerevisiae, Schizosaccharomyces pombe, Pichia pastoris*), cell lines of mammals (for example CHO, HEK, COS) or insects (for example Sf9, Sf21).

There is an option of modifying the polynucleotide sequences used in the heterologous expression of polypeptides to give rise to polypeptides modified by means of deletions, insertions and/or substitutions in their amino acid sequence although they continue to maintain their immunogenic capacity for the purpose of obtaining variations to make their expression and/or purification easier, or to increase their plasma half-life, increasing their therapeutic efficacy, and reducing the degree and/or the onset of side effects, if their use is therapeutic.

The introduction of the genetic material into the host cell or expression system of the polypeptide is performed, for example, by transfection with calcium salts, spheroplasts, electroporation, liposomes or viral vectors. With respect to the vector for the inclusion of the genetic material inside the cell, there is a possibility of using any of the conventional expression vectors developed for the expression of proteins in prokaryotic and eukaryotic cells.

Once the recombinant polypeptides obtained are purified, they are used in obtaining antibodies, for the development of diagnostic tests and for the preparation of immunogenic pharmaceutical compositions.

### Polynucleotides

In another aspect, the disclosure also comprises polynucleotides obtained from the isolated parasite of the invention. In this description the term "nucleic acid or polynucleotide" relates to molecules of the DNA or RNA nucleic acids obtained from the isolate of the invention in any stage of its biological cycle.

Likewise, the polynucleotides of the disclosure include "identical" sequences and also sequences "substantially identical" thereto. In fact, a variable number of polynucleotide sequences which are transcribed and translated can produce identical functional polypeptides due to the degeneration of the genetic code. As discussed in the previous section, polynucleotide sequences are "substantially identical" when they have at least 60%, preferably 80% and much more preferably between 90-95% of identity in the nucleotide residues, when they are compared, aligned for maximum correspondence and their percentage of identity calculated by the already mentioned methods. Two polynucleotide sequences are also substantially identical if two molecules hybridize one with the other under high specificity conditions. These conditions depend on the sequence and will be different depending on the assayed conditions. The selected conditions are generally the temperature, which is usually up to 5°C lower than the dissociation temperature or Tm for a specific sequence in a saline solution at a determined pH. The Tm is the temperature at which 50% of the sequence was dehybridized from the complementary sequence.

One of the applications of these molecules polynucleotide comprises the heterologous expression of proteins of *Neospora* of diagnostic or immunogenic interest, as previously described, for the preparation of DNA vaccines or as probes in different diagnostic tests of the infection in animals from a biological sample.

The polynucleotides of interest in the development of diagnostic tests and for the expression of recombinant proteins are obtained by means of techniques known in the art and, preferably, by means of the polymerase chain reaction or PCR technique from messenger RNA, genomic DNA or gene libraries. One option for the identification of the polynucleotide required for the heterologous expression of a specific protein is based on reverse translation and on the design of oligonucleotides degenerated from the sequence of the protein of interest or of the conserved regions of homologous proteins of phylogenetically related species. Once identified, new oligonucleotides are designed based on its sequence, which will be used for the amplification and cloning of the polynucleotide of interest for their subsequent heterologous expression, the diagnosis of disease by means of the amplification of specific *Neospora* DNA from the DNA obtained from a biological sample, or for obtaining nucleotide probes which allow detecting the presence of messenger RNA or DNA of the parasite in clinical samples, or for any other method of detection based on the nucleic acids of the parasite.

### Use of the isolate of the invention in immunogenic compositions

The invention also includes the use of the new isolate for preparing a vaccine for the treatment and/or prevention of the infection caused by *N. caninum* in animals.

These preparations are formulated from the isolate of the invention in any of the stages of its biological cycle, preferably from the attenuated live, inactivated and/or fixed parasite, or they are formed by a cell extract or lysate thereof.

As already described, another aspect of the disclosure relates to the use of partially or completely purified antigenic proteins, recombinant or not, for the preparation of vaccine products. The vaccines included in the disclosure also include recombinant vaccines such as DNA vaccines by means of cloning the gene encoding the immunogen in plasmid vectors which are transfected into the host cells in the animals. The virus, such as, for example, the vaccine virus (*vaccinia*) or some adenovirus as vectors, are other alternatives for the generation of recombinant vaccines.

The vaccines formed by the different immunogens described are preferably prepared as injectables in liquid formulations, such as solutions, suspensions or emulsions in which the immunogen is encapsulated or not in liposomes or any other suitable system, or in solid form for their subsequent reconstitution prior to its administration. However they can also be presented as oral formulations (for example capsules and tablets).

In its formulation the immunogen can be combined with excipients for pharmaceutical use, wetting agents or emulsifiers, pH regulators, preservatives and/or adjuvants increasing the effectiveness of the vaccine, in addition to cytokines and/or other immunomodulators.

The vaccines of the present invention can be administered preventively to animals sensitive to infection for the purpose of inducing an effective immune response against the parasite. The vaccine can also be administered for the treatment of the disease once the first clinical signs are shown.

The use of antibodies that specifically react against an antigen from the isolate of the invention, and preferably those having a neutralizing activity against the parasite in passive immunotherapies, for the preparation of a pharmaceutical product for the treatment of the infection and/or disease caused by *N. caninum* in the animals, is also part of the present disclosure.

### Use of the isolate of the invention in diagnostic techniques

In this description the term "biological sample or clinical sample" relates to any sample obtained from living or dead organisms. Examples of biological samples are biological fluids (blood, serum, plasma, milk, urine, semen, ascitic fluid, cerebrospinal fluid and fetal fluids), tissue samples (from the brain, spinal cord, lung, heart, liver and placenta, among others) and cell cultures.

The use of antigens from the isolate of the invention to detect infection by the *N. caninum* parasite in animals from a biological sample is included as an object of the disclosure.

The antigen will preferably be formed by the isolate itself in any of its stages of the biological cycle, extracts or cell lysates obtained therefrom, antigenic polypeptides obtained from the isolate of the invention or produced by means of recombinant DNA techniques from partially or completely purified polynucleotides obtained from the isolate of the invention, or mixtures thereof.

The antigen used will more preferably be formed by tachyzoites and/or bradyzoites, and antigenic polypeptides.

The use of antibodies that specifically react against an antigen from the isolate of the invention to detect infection by the *N. caninum* parasite in animals from a biological sample is also part of this disclosure. Even though the antibodies can be monoclonal antibodies or can be formed by a polyclonal mixture, a preferred embodiment of this disclosure uses monoclonal antibodies.

The diagnosis of the infection by the *Neospora* parasite in animals from a biological sample can be performed by means of different immunoassay techniques allowing the detection of antibodies specific for *N. caninum* generated during the immune response developed in the infected animal, such as immunoenzymatic (such as the ELISA technique), immunofluorescence (such as the IFA technique), immunoblot (IB) and agglutination (DAT) techniques or any other method based on the detection of the mentioned antibodies.

For diagnostic purposes, the detection of the parasite in a biological sample can also be performed by means of immunological techniques using antibodies specific for the parasite (such as the immunohistochemical (IHC) technique).

This disclosure also includes the detection of the nucleic acids of the parasite for diagnostic purposes by means of the use of different molecular biology techniques, based on specific hybridization techniques, stressing the application of PCR, *in situ* hybridization and *in situ* PCR. In this description the expression "specific hybridization" relates to the specific hybridization between the sequence acting as a probe with the target sequence. Although the probe could bind to other unrelated sequences, at least 90% and preferably 95% or more of the hybridization complexes formed will be with the target sequence.

Furthermore, it is possible to perform the *in vivo* and *in vitro* isolation of the parasite from a biological sample in systems, and its subsequent identification by means of any of the already mentioned techniques.

### Uses of the genetically modified isolate of the invention

The disclosure also includes the use of the new genetically modified isolate and of the extracts obtained therefrom for the preparation of diagnostic tests and the preparation of products for the treatment and prevention of the infection caused by *Neospora.* Additionally and with the same uses, the polypeptides and polynucleotides obtained from the genetically modified isolate and the immunoreactive antibodies developed against antigens thereof are included.

The isolate of the invention can be genetically manipulated by means of the insertion and/or deletion of homologous or heterologous polynucleotide sequences in its genome, or by means of directed mutagenesis by the substitution of nucleotides in the sequence of the genome thereof. There is an option of modifying the homologous or heterologous polynucleotide sequences selected for their insertion in the genome of the isolate by means of deletions, insertions and/or substitutions in its nucleotide sequence for the purpose of achieving and making their expression in the different stages of the biological cycle of the parasite easier. The introduction of the genetic material is performed by means of processes well known in the art such as the electroporation or the transformation with calcium salts. The isolate can also be genetically modified by alternative processes including random mutagenesis using chemical agents, such as ethyl methanesulfonate (EMS) or diethyl sulfate (DES) and physical agents such as ionizing and/or ultraviolet radiations.

The genetically modified isolates of the disclosure mainly have a diagnostic and immunoprophylactic interest. In the first case, they are part of immunoassay tests incorporating the expression of new antigenic proteins which allow increasing the sensitivity and specificity of the assay for the detection of antibodies specific for *N. caninum* (for example, expression of specific proteins of bradyzoite during the tachyzoite stage used as an antigen), and in the second case, they are part of vaccines for the prevention and treatment of infections caused by *Neospora* by means of the generation of isolates expressing new antigenic proteins of immunoprophylactic interest (specific of the bradyzoite and/or sporozoite stages), by means of the generation of isolates attenuated by the genetic modification of genes directly involved in the virulence of *Neospora* or the insertion of polynucleotide sequences as "markers" if the isolate is part of a live vaccine.

### Description of the Drawings

To complement the description and for the purpose of aiding to better understand the features of the invention, a set of drawings is attached as an integral part of the present specification, in which the following is depicted with an illustrative and non-limiting character:
Figure 1 shows the parasite load present in the lung (panel A) and brain (panel B) in the experimental murine infection model developed with the isolate Nc-Spain 7 in Example 3 of this specification.
Figure 2 shows the assessment of the humoral immune response in the experimental murine infection model developed with the isolate Nc-Spain 7, as explained in Example 4 of this specification.
Figure 3 shows the percentage of *in vitro* conversion into bradyzoite stage of tachyzoites of the isolate Nc-Spain 7.
Figure 4 shows the assessment of the humoral immune response developed by the different groups of animals of the vaccine test developed with the isolate Nc-Spain 7.
Figure 5 shows the immunoblot analysis with the whole protein extract of the isolates of *N. caninum,* Nc-Spain 7 (Panel A) and Nc-1 (Panel B), under reducing conditions with a mixture at equal parts of the plasmas obtained from experimentally infected mice.
Figure 6 shows the immunoblot analysis with the whole protein extract of the isolates of *N. caninum,* Nc-Spain 7 (Panel A) and Nc-1 (Panel B), under reducing conditions with sera obtained from bovines.

### Embodiment of the Invention

The following examples are set forth for the purposes of providing the person skilled in the art with a sufficiently clear and complete explanation of the present invention, but they must not be considered as limitations on the essential aspects of the object thereof as they have been set forth in the previous sections of this specification.

### Example 1.- Obtaining a new isolate of N. caninum from a congenitally infected calf

This example describes obtaining the isolate of *N. caninum* called Nc-Spain 7 from a congenitally infected calf of the Holstein Friesian race.

The calf was born from a cow with positive serology in the detection tests for antibodies specific for *N. caninum* maintained in a milk production farm located in the province of Navarre, Spain. The precolostral serum of the calf was analyzed for the detection of antibodies specific for *Neospora* by the IFA technique, resulting in a titer of 1:800. Two months after its birth it remained healthy and showing no clinical sign attributable to infection by *Neospora.* It was sacrificed on this date by means of an injection of 30 ml of T-61 (embutramide and mebezonium iodide, Intervet) by endovenous route and the brain and the upper portion of the spinal cord were then immediately extracted under aseptic conditions and maintained at 4°C.

After the necropsy, different portions of 5 to 15 grams of weight of both hemispheres and of the anterior, mid and posterior region of the brain, as well as of the cerebellum, brain stem and the spinal cord, were removed under aseptic conditions and were maintained at 4°C in a PBS buffer solution supplemented with an antibiotic-antimycotic solution (penicillin G 200 IU/ml, streptomycin 200 µg/ml and amphotericin B 500 ng/ml) (Gibco, BRL, UK) until their subsequent inoculation in mice of the athymic Balb/c *nu*/*nu* strain. Simultaneously, different portions of brain from the same locations were fixed in a 10% buffered formalin solution for their subsequent histological analysis.

Before performing the isolation of the parasite, the different samples of nervous tissue maintained at 4°C were analyzed for the purpose of detecting the presence of the DNA of *N. caninum* by means of the use of nested PCR amplifying the ITS-1 region of the parasite (Buxton et al., 1998, J. Comp Pathol. 118: 267-279). To that end, different portions of about 20 mg were removed from all the sections collected and processed for the extraction of the genomic DNA with the commercial kit "Genomic-Prep cell and tissue DNA isolation kit" (Amershan Biosciences) following the manufacturer's instructions, and subsequently 5 µl of the solution of the resulting DNA (100-200 ng) were used as a mold in the nested PCR of the ITS-1 region described below.

The amplification of the ITS-1 region of the ribosomal DNA of *N. caninum* was performed in a first PCR reaction using the oligonucleotides NN1 (SEQ ID NO: 13) and NN2 (SEQ ID NO: 14), and in a second reaction using the oligonucleotides NP1 (SEQ ID NO: 15) and NP2 (SEQ ID NO: 16). The PCR mixture (25 µl) was made up of 0.15 µM of each oligo (Sigma), 200 µM dNTPs (Sigma), 1x buffer (75 mM Tris pH 9, 20 mM (NH₄)₂SO₄, 50 mM KCI) (Biotools, Spain), 2 mM MgCl₂ (Biotools) and 0.625 units of DNA polymerase (Biotools). In the first amplification reaction 5 µl of DNA and in the second 2 µl of the product of the first reaction were added. Both reactions were performed in a thermocycler according to the following program: 94°C for 5 minutes and 25 cycles of 94°C/1 minute, 48°C/1 minute and 72°C/1.5 minutes and a final cycle of 72°C for 5 minutes. The products resulting from the second amplification, with a size of 213 bp, were viewed in 1.6% agarose gel in 0.5 x TBE (Tris-borate-EDTA buffer) stained with ethidium bromide.

DNA of the parasite was detected in all the analyzed brain sections.

Furthermore, the samples maintained in the 10% buffered formalin solution were dehydrated, embedded in paraffin and multiple sections of 3 to 6 µm thick were stained with hematoxylin-eosin following the usual method. For the detection of lesions compatible with the infection of *N. caninum* the samples were microscopically examined as described in González et al., 1999, Vet Rec 144: 145-150. All the analyzed samples showed lesions compatible with the infection by this parasite characterized by symptoms of non-suppurative meningoencephalitis, with multiple glial nodules and perivascular cuffing. Likewise, sections of several of the blocks were deparaffinized and examined by means of the immunohistochemical technique as described in Pereira-Bueno et al., 2003, Vet Parasitol 111: 143-152, using a rabbit polyclonal serum developed against tachyzoites of *N. caninum,* as well as a rabbit polyclonal serum against the protein BAG1 as described in Example 5 of the present specification. The presence of structures compatible with cysts formed by this parasite was detected in most of the analyzed sections.

Once the presence of the parasite was confirmed, the brain samples maintained at 4°C were processed for isolation by means of their homogenization with cycles of 1 minute of duration in a tissue homogenizer (IUL Masticator) until their perfect breakdown. Then the filtration of the macerated products through a previously sterilized 150 µm mesh was performed, and it was centrifuged at 1350 x *g* for 10 minutes. The supernatant was discarded and the sediment equivalent to about 5 grams of starting tissue was intraperitoneally inoculated with an insulin syringe and needle with a diameter of 0.5x16 mm in female mice of the Balb/c *nu*/*nu* strain with an approximate weight of 20-25 grams. The entire process was performed under aseptic conditions.

The inoculated mice were examined daily to detect the onset of clinical signs attributable to neosporosis, including weight loss, limb paralysis, ataxia, lethargy, and other neurological signs, as described in Yamane et al., 1998, J. Vet. Diagn. Invest. 10: 364-368. These signs were detected in all the inoculated mice between the days 18-21 post-inoculation (PI) and, after their onset, the mice were sacrificed in CO₂ atmosphere and used for the isolation of the parasite in cell culture by means of the inoculation of the peritoneal wash.

Furthermore, for the purpose of maintaining the isolate *in vivo* in the murine model, the brain of the sacrificed mice was removed and homogenized in a PBS solution supplemented with an antibiotic-antimycotic solution (100 IU/ml penicillin G, 100 µg/ml streptomycin and 250 ng/ml amphotericin B) (Gibco, BRL, UK) by means of successive syringe passages with 21 G and 25 G needles and, then the homogenate equivalent to the fourth part of the brain, was intraperitoneally inoculated in a new athymic mouse of the Balbc *nu*/*nu* strain which was likewise monitored until the onset of the previously described clinical signs. The process was performed up to 6 consecutive passages from mouse to mouse, observing the onset of the already described clinical signs, in all the inoculated mice, between days 6 and 15 PI. For the purpose of confirming that the observed clinical signs were a consequence of the infection by *N. caninum,* the extraction of the DNA from 20 mg of sample of the brain of the mice inoculated with the brain of the calf was performed, which was subjected to analysis by means of the nested PCR of the previously described ITS-1 region, the specific amplification product being detected in all the analyzed mice.

With respect to the isolation in cell culture, it was performed by means of the inoculation of the peritoneal wash of the previously sacrificed mice with clinical signs on a monolayer culture of the cell line MARC-145. To that end, the washing of the peritoneal cavity was performed with approximately 8 ml of Dulbeco's Modified Eagle Medium (DMEM) supplemented with 2% bovine fetal serum and a 2% antibiotic-antimycotic solution (200 IU/ml penicillin G, 200 µg/ml streptomycin and 500 ng/ml amphotericin B) (Gibco BRL, UK) which was then immediately added to a monolayer of approximately 10⁶ cells of the line MARC-145 in a culture flask of 25 cm².

After 24 hours, the medium was removed and substituted with DMEM supplemented with 2% equine serum and the same 1% antibiotic-antimycotic solution. Every 4 days the layer of cells was mechanically lifted with the use of a cell scraper, it was passed through a syringe and 25 G needle and added to a new culture of 5x10⁵ MARC-145 cells. After 24 hours the medium was removed again and substituted with DMEM supplemented with 2% equine serum.

The cultures were maintained in a wet oven at 37°C and 5% CO₂ atmosphere and were observed daily under phase contrast microscopy for viewing the parasite. At 4 days PI some vacuoles and tachyzoites were observed in the cell culture, which was then maintained by successive passages according to the described process until obtaining counts in a Neubauer chamber equal to or greater than 10⁶ tachyzoites at the third passage. From this moment, the culture was maintained as described by Perez-Zaballos et al., 2005, J. Parasitol., 91 (3): 507-10.

### Example 2.- Genetic characterization of the isolate Nc-Spain 7

This example describes the genetic characterization of the isolate Nc-Spain 7 along with other isolates obtained from different hosts and from a different geographic origin, allowing their identification and discrimination from the rest. To that end, 13 microsatellite sequences previously identified in the genome of *N. caninum* were amplified and sequenced from the isolate of the invention, as had previously been done with 9 other isolates (Regidor-Cerrillo et al., 2006, J. Parasitol., 92(3): 517-524).

The microsatellites are formed by single nucleotide sequences of 2 to 5 nucleotides which are repeated in tandem and which are characterized by elevated polymorphism, mainly depending on the number of repeats of the nucleotide motif they have, being a suitable tool for the identification and discrimination of the different isolates of *N. caninum.*

For the identification of microsatellite sequences in the genome of *N. caninum,* approximately 25,330 ESTs (Expression Sequences Tags) deposited in public databases derived from the isolates of *N. caninum,* Nc-1 and Nc-Liv, were analyzed with the Tandem Repeat Occurrence Locator (TROLL) (Castelo et al., 2002. Bioinformatics. 18:634-636), Tandem Repeats Finder (TRF) (Benson 1999, Nucleic Acids Res. 27:573-580) computer programs and, finally, to prevent the analysis of redundant sequences, by means of the BLASTN computer program.

13 markers of the 126 microsatellite sequences detected were selected depending on several criteria which included the possibility of designing primers for their subsequent PCR amplification and sequencing, the number of tandem repeats they had, selecting those with the highest number, and their location outside encoding regions of the genome, for the purpose of selecting the most polymorphic. Table I describes the 13 microsatellites used in this study as molecular markers. For each selected microsatellite, which were called as specified in Table I, specific primers (SEQ ID NO: 17 to SEQ ID NO: 42) were designed which were used for the amplification of PCR products of approximately 300 bp, including the repeat motif and part of the sequences which flank it, from the genomic DNA of ten isolates of *N. caninum* of different hosts and geographic origin, including the isolate object of the invention, as shown in Table II.

The genomic DNA of the isolate Nc-Spain 7 was obtained from 10⁸ tachyzoites by means of the use of the Genomic Prep Cells and Tissue DNA Isolation Kit commercial system (Amersham Biosciences, Uppsala, Sweden). To that end, the tachyzoites maintained in monolayer of cell line MARC-145, as described in Example 1, were collected from the culture when 70-80% of the parasitophorous vacuoles had been ruptured and purified from the cell residues by means of several passages through a 25 G needle and the use of PD10 chromatograph columns (Amersham Biosciences, Uppsala, Sweden). After their extraction, the concentration of the genomic DNA was determined by means of spectrophotometry at 260/280 nm and stored at - 20°C.

The PCR reactions were performed in volumes of 50 µl made up of 0.2 µM of each oligonucleotide, 200 µM dNTPs (Sigma), 1x reaction buffer (75 mM Tris pH 9, 20 mM (NH₄)₂SO₄, 50 mM KCI,) (Biotools, Spain), 2 mM MgCl₂ (Biotools), 2 units of DNA polymerase (Biotools) and 50 ng of DNA mold. The reaction conditions were the following: 1 cycle at 94°C for 5 minutes, 35 cycles of 94°C/1 minute, 60°C/1 minute, and 72°C/1 minute, with a final cycle of 72°C for 10 minutes. In each series of amplifications, DNA obtained from the cells in which the parasite was usually maintained and H₂O were included as a negative control. The PCR products obtained were separated by electrophoresis in agarose gels with 2.5% ethidium bromide (NuSieve 3:1, Cambrex BioScience, USA) and were viewed under UV light. The products were subsequently purified with the Exo-SAP IT kit (USB, USA), according to the manufacturer's instructions, and directly sequenced in both directions. The sequences were analyzed using the BioEdit Sequence Alignment Editor v.7.0.1 computer program (Copyright© 1997-2004 Tom Hall, Ibis Therapeutics, Carlsbad CA 92008, USA) and were deposited in the GenBank database under access numbers AY935165-AY935200 and AY937107-AY937178, except for the isolate Nc-Spain 7.

The presence of polymorphism for the 13 microsatellites selected ranged from a low polymorphism with 2 or 3 alleles for each microsatellite (MS1A, MS1B, MS12 and MS21) to 4 to 9 alleles for the remaining microsatellites in the 10 isolates analyzed. Furthermore, MS7 showed an allele carrying an SNP (Single Nucleotide Polymorphism) in the microsatellite. The sequences obtained for the isolate object of the invention are described in SEQ ID NO: 1 (microsatellite MS1A), SEQ ID NO: 2 (microsatellite MS1B), SEQ ID NO: 3 (microsatellite MS2), SEQ ID NO: 4 (microsatellite MS3), SEQ ID NO: 5 (microsatellite MS4), SEQ ID NO: 6 (microsatellite MS5), SEQ ID NO: 7 (microsatellites MS6A and MS6B), SEQ ID NO: 8 (microsatellite MS7), SEQ ID NO: 9 (microsatellite MS8), SEQ ID NO: 10 (microsatellite MS10), SEQ ID NO: 11 (microsatellite MS12), SEQ ID NO: 12 (microsatellite MS21).

For the multilocus analysis of the genotypes of each isolate, each allele was designed with a number according to the length of the repeat motif and the differences found in its sequence. The multilocus analysis considerably increased the discriminatory power of the microsatellite sequences and unique genetic profiles were thus obtained for each isolate, indicating that all the isolates significantly differed from one another, as can be seen in Table II.

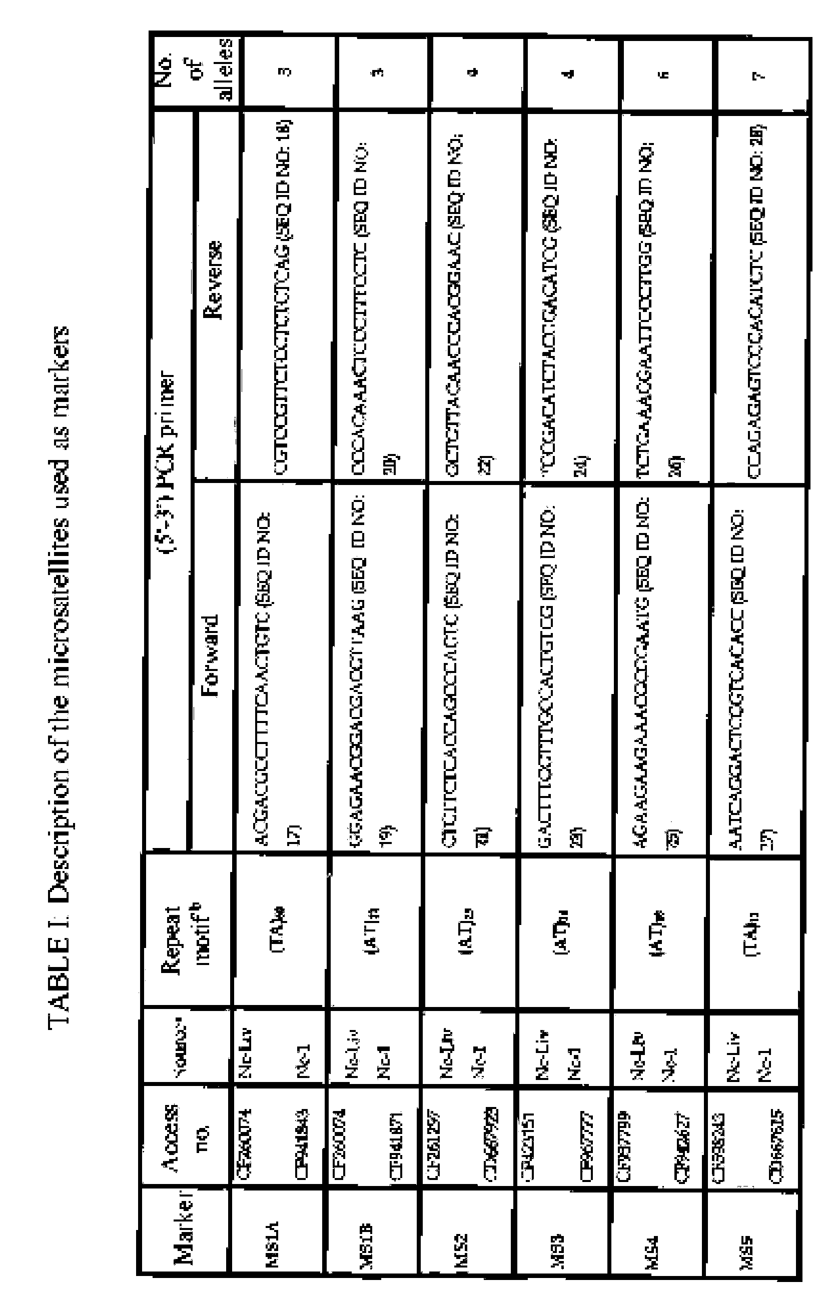

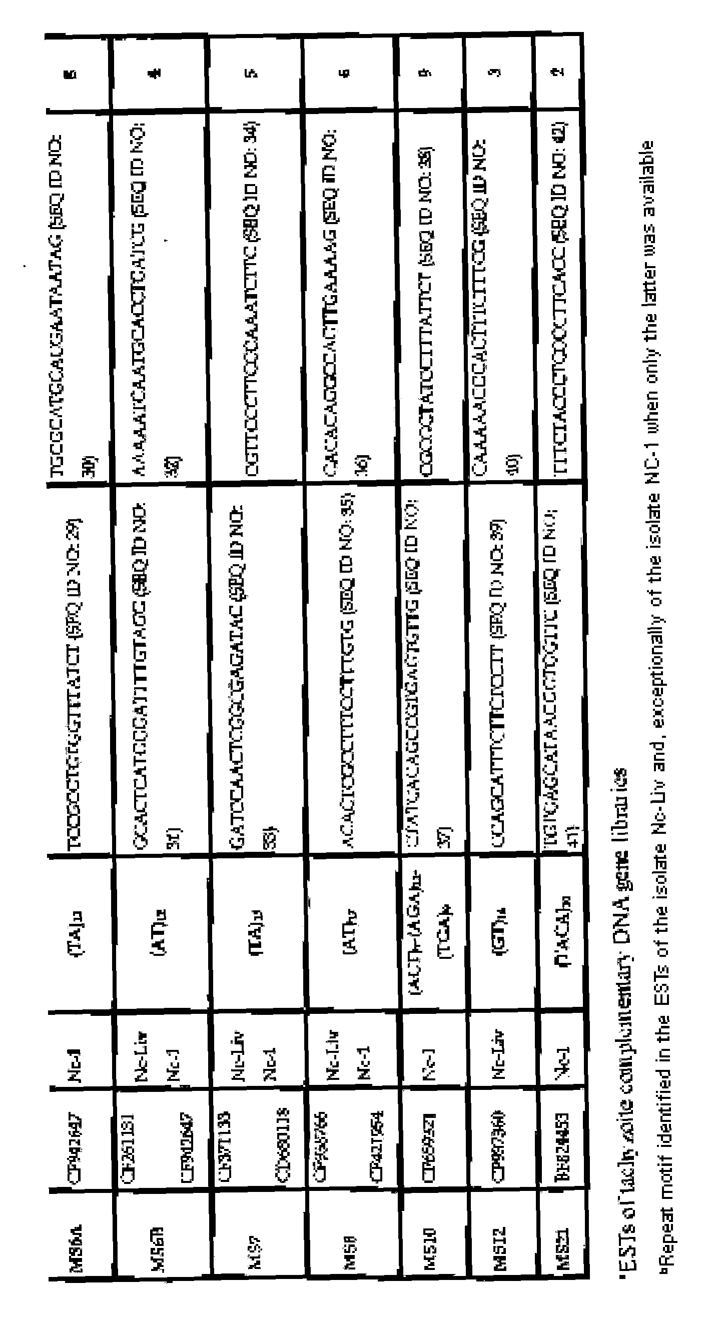

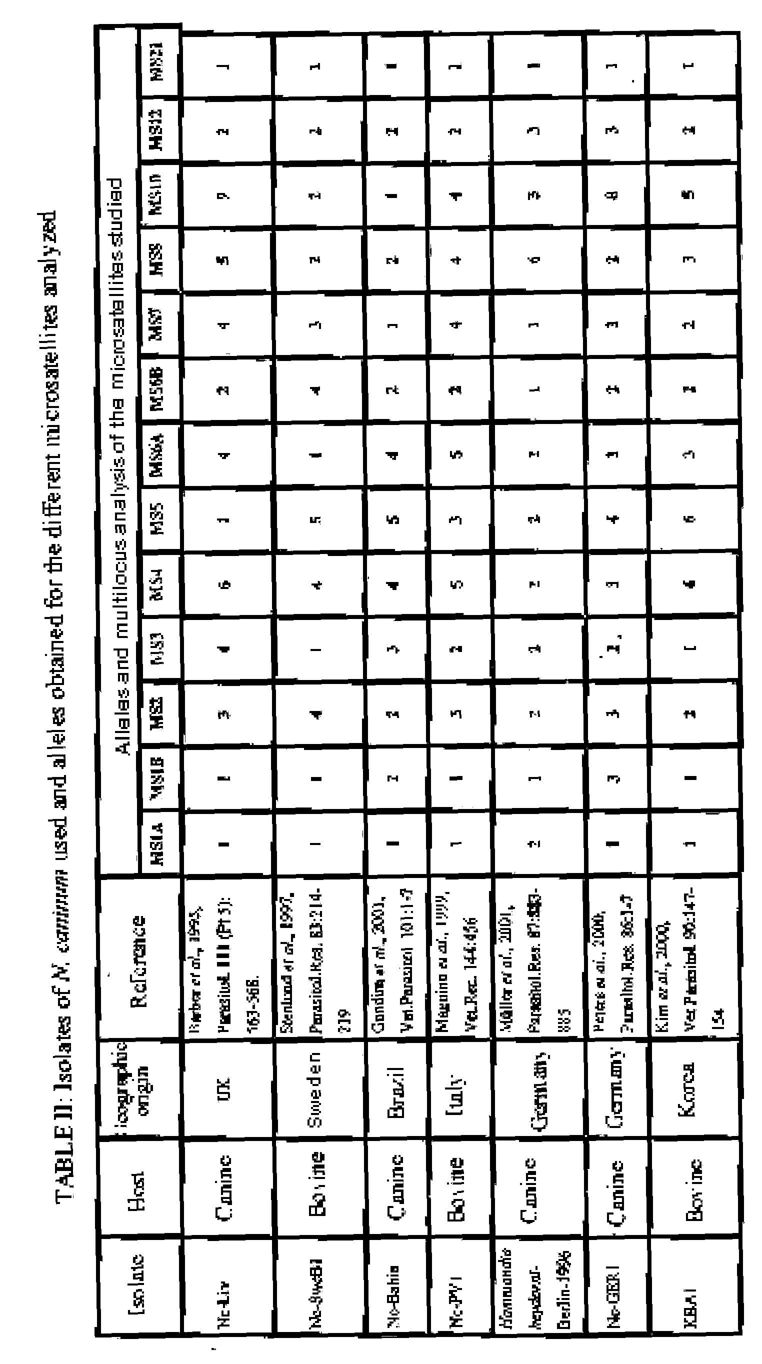

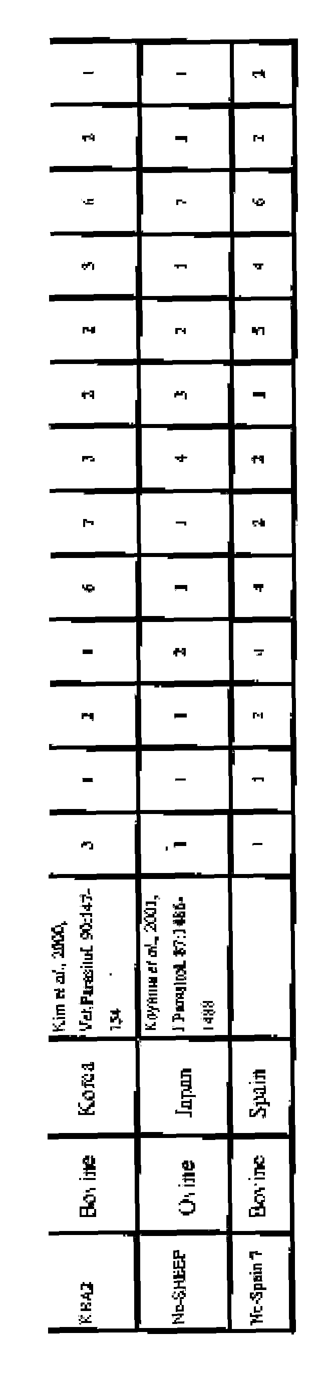

### Example 3.- Pathogenic characterization of the isolate Nc-Spain 7 in a murine model

This section describes the experiments conducted to determine the pathogenicity of the isolate object of the invention Nc-Spain 7 in an experimental murine model.

Female mice of the BALB/c strain of 6 weeks of age were inoculated by intraperitoneal route with 10⁶ (group A) and 10⁷ (group B) tachyzoites of the isolate Nc-Spain 7 maintained in cell culture as described in Example 1. The control group (group C) consisted of BALB/c mice of the same features inoculated with 200 µl of PBS by intraperitoneal route. Five animals from each group were sacrificed randomly in the course of the experiment on days 1, 2, 4, 8, 16, 32 and 64, except for the control group, which were sacrificed three mice a day.

All the mice were examined daily to observe clinical signs compatible with the infection by *N. caninum.*

The mice were sacrificed in CO₂ atmosphere. Immediately after the sacrifice blood was obtained by means of intracardial puncture with an insulin syringe and 0.5 x 16 mm needle. The mice were weighed at the time of the sacrifice for the purpose of detecting weight reductions with respect to the control group. The blood samples (200-500 µl) were deposited in 1.5 ml tubes with EDTA for the parasitemia study. Subsequently, the necropsy of each subject was performed, opening the abdominal and chest cavities, assessing the presence of macroscopic lesions and removing the lung and the brain, which were frozen at -80°C in sterile 1.5 ml tubes until extraction of the DNA for the purpose of detecting the presence of the parasite by PCR.

The blood samples were centrifuged at 2000 x g for 10 minutes. The sediment formed by the blood cells was intended for the extraction of the DNA using the "Genomic-Prep cell and blood DNA isolation kit" commercial system (Amershan Biosciences) and the plasma obtained was maintained at -80°C. The lung and brain DNA was extracted from 10-20 mg of frozen tissue with the Genomic-Prep cell and tissue DNA isolation kit commercial system (Amershan Biosciences).

For the detection of the parasite in the blood, lung and brain samples collected from the sacrificed animals, the nested PCR technique of the ITS-1 region was used, following the process described in Example 1. In all the reactions the DNA obtained from tachyzoites of *N. caninum* was used as a positive control and sterile ultra-pure water was used as a negative control.

All the mice of the control group (group C) survived until the end of the experiment clinically healthy. In contrast, one of the mice of group A developed clinical signs clearly compatible with neosporosis (hemiparesis and ataxia) from day 31 and survived until day 38 post-inoculation (PI) when, due to the severity of the symptoms, it was sacrificed. In group B a total of 11 mice presented clinical signs compatible with neosporosis to a greater or lesser degree throughout the experiment. From day 14 PI, they presented bristling of the hair, severe anorexia, lethargy, hemiparesis and some of them, staggers. Three of the mice were sacrificed on days 24 and 58 PI due to the severity of the symptoms.

Splenomegaly was observed during the necropsy in most of the mice sacrificed of groups A and B from day 2 to day 16 PI, and lymphadenopathy in mesenteric and iliac nodes from day 2 until day 32, in group A, and from day 1 until the end of the experiment in group B.

The DNA of *N. caninum* was detected in blood from day 1 to 8 PI, and in the lung and brain from day 2 to 64 PI. With respect to the frequency of detection of *N. caninum,* a higher detectability of the parasite was observed when the dose of the inoculum was increased. In group C (control group), all the analyzed samples were negative, as was expected. The results obtained for the distribution of the parasite in the groups A and B are shown in Table III.

In said Table it can be observed that from 16 days PI the parasite disappears from the blood and from the lungs of the mice, even from those that were inoculated with the highest dose. With respect to the presence of the parasite in the brain, the DNA was detected from day 16 PI in group A and from day 4 PI in group B until day 64 PI, demonstrating the stationing capability of the isolate of the infection in this organ.

**TABLE III: Detection of the DNA of the isolate Nc-Spain 7 in blood, lung and brain of mice throughout the experiment of Example 3 in groups A and B**

| | **Blood** | **Lung** | | | **Brain** | |
|---|---|---|---|---|---|---|
| Days PI | Group A | Group B | Group A | Group B | Group A | Group B |
| 1 | 0/5^{a} (0%)^{b} | 3/5 (60%) | 0/5 (0%) | 0/5 (0%) | 0/5 (0%) | 0/5 (0%) |
| 2 | 1/5 (20%) | 3/5 (60%) | 0/5 (0%) | 2/5 (40%) | 0/5 (0%) | 0/5 (0%) |
| 4 | 1/5 (20%) | 1/5 (20%) | 2/5 (40%) | 5/5 (100%) | 0/5 (0%) | 1/5 (20%) |
| 8 | 0/5 (0%) | 2/5 (40%) | 4/5 (80%) | 5/5 (100%) | 0/5 (0%) | 4/5 (80%) |
| 16 | 0/5 (0%) | 0/5 (0%) | 0/5 (0%) | 3/5 (60%) | 2/5 (40%) | 5/5 (100%) |
| 24^{c} | - | 0/2^{c} | - | 0/2^{c} | - | 2/2^{c} |
| 32 | 0/5 (0%) | 0/4 (0%) | 0/5 (0%) | 0/4 (0%) | 2/5 (40%) | 4/4 (100%) |
| 38^{c} | 0/1^{c} | - | 0/1^{c} | - | 1/1^{c} | - |
| 58^{c} | - | 0/1^{c} | - | 0/1^{c} | - | 1/1^{c} |
| 64 | 0/5 (0%) | 0/4 (0%) | 0/5 (0%) | 0/4 (0%) | 4/5 (80%) | 4/4 (100%) |

| | | | | | | |
|---|---|---|---|---|---|---|
| ^{a} The fractions represent the number of mice positive from the total number of mice. ^{b} Percentage of detection. ^{c} Animals sacrificed due to the severity of their symptoms. | | | | | | |

The parasite load of the positive brain and lung samples was determined by means of quantitative real-time PCR technique as described in patent EP1655378.

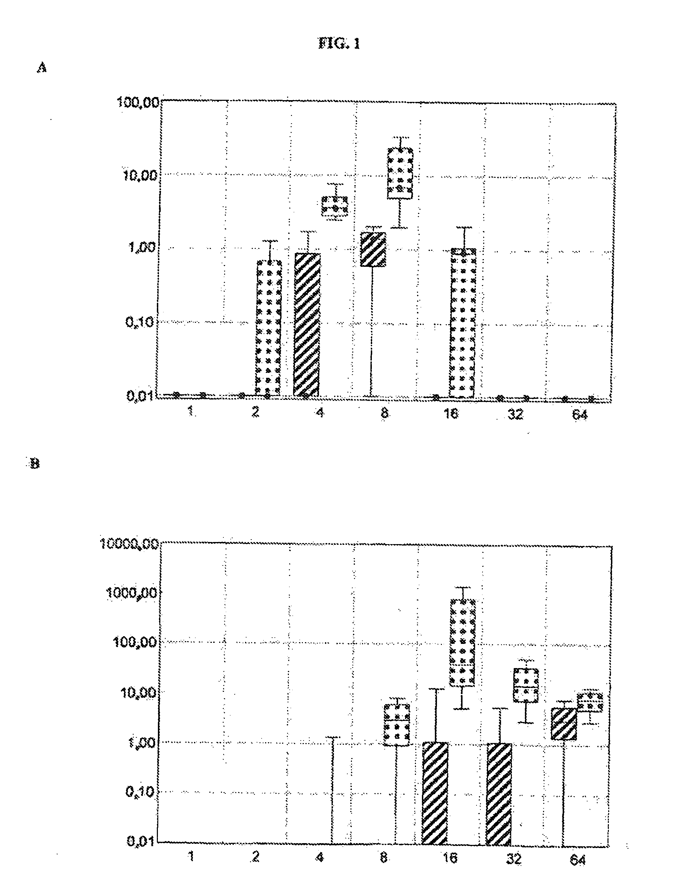

The parasite loads obtained for group A in the lung were low on day 4 PI, slightly increasing on day 8 PI. In the case of group B, the detected load was significantly increased on day 4 PI, reaching a maximum on day 8 PI (Figure 1, panel A). When the parasite loads in the brain of the mice of group A were studied, the number of parasites found on days 16 and 32 PI was similar and slightly increased on day 64 PI. In group B the parasite load was maximum on day 16 PI, and on days 32 and 64 it decreased, although it was maintained at elevated levels. The comparative statistical analysis of both groups by means of the Statistica computer program (version 5.0, copyright Statsoft. Inc.) demonstrated that during days 16 and 32 PI, group B had a higher parasite load (P < 0.05 Mann-Whitney U), however on day 64 PI, the detected loads in both groups were similar (P > 0.05 Mann-Whitney U) (Figure 1, panel B).

In Figure 1, the y-axis represents the number of tachyzoites detected per microgram of genomic DNA of the host, quantified by the real-time PCR technique in the lung (Panel A) and brain (Panel B) of the mice of groups A (point bars), inoculated with 10⁶ tachyzoites of the isolate Nc-Spain 7, and B (striped bars), inoculated with 10⁷ tachyzoites, throughout the experimental infection. The day post-inoculation is represented on the x-axis. The bars are represented with their median and the 25% and 75% quartiles.

### Example 4.- Immunogenicity of the isolate Nc-Spain 7 in a murine model

The detection of the immunoglobulins of the isotypes IgG1 and IgG2a specific for *N. caninum* in the plasma of the infected mice of Example 3 was performed by means of an indirect immunoenzymatic assay (ELISA).

To that end, each 96-well plate (Nunc Maxisorp) was antigenized with 0.5 µg of protein of the soluble extract of tachyzoites of the isolate Nc-1 of *N. caninum* obtained by sonication and fixed with 10% buffered formalin solution. After blocking 0.05% PBS-Tween-20 buffer and 1% bovine serum albumin and incubating with the test sera at a 1/100 dilution, anti-IgG1 and anti-IgG2a of mice conjugated with peroxidase (Southern Biotechnology) at a 1/5000 dilution were used as secondary antibodies. The colorimetric reaction was finally developed by means of the addition of the o-dihydrochloride-diamino-phenylene substrate (0.6 mg/ml; Sigma) in 0.05 M citrate buffer supplemented with 0.045% H₂O₂. After stopping the colorimetric reaction with a solution of 2M H₂SO₄, the absorbance was measured in an ELISA reader at a wavelength of 405 nm. The results were expressed as optical density. A sample of negative mouse serum (optical density less than 0.1) as well as a mouse serum experimentally infected with an optical density greater than 2.0 were used as controls of the test.

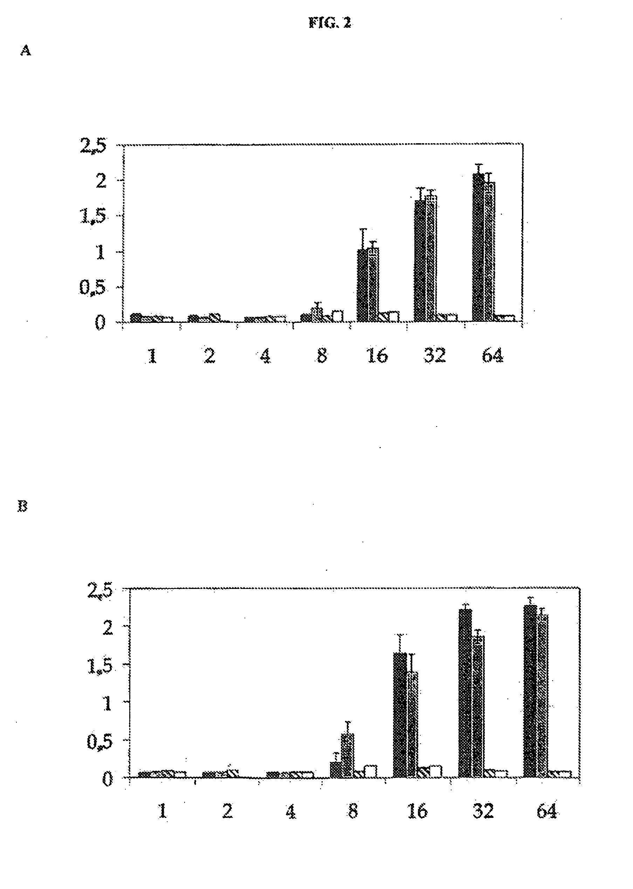

The production of immunoglobulins IgG1 and IgG2a was not detected until day 8 PI for both groups A and B. In group A, after day 16 PI an intense increase of the levels detected for both isotypes occurred, which was maintained until day 64 PI. Throughout the experiment, the mice inoculated with 10⁶ tachyzoites had a concentration of IgG2a slightly greater than IgG1 for days 8, 16 and 32 PI, although on day 64 PI the concentration of IgG1 slightly exceeded that of IgG2a (Figure 2, panel A). With respect to group B, an increase of the levels of both immunoglobulins occurred after 8 PI which was maintained until day 64 PI, in which maximum values were obtained. In this case the production of IgG2a on day 8 PI was considerably greater than that of IgG1, whereas on days 16, 32 and 64 PI the levels of IgG1 predominated compared with those of IgG2a (Figure 2, panel B).

In Figure 2, the optical density (OD), determined at 450 nm using the ELISA technique, is represented on the y-axis as the measurement of antibodies IgG1 (black bars) and IgG2a (grey bars) specific for *N. caninum* produced by the mice of groups A, (Panel A) and B (Panel B), which were inoculated with 10⁶ and 10⁷ tachyzoites of the isolate Nc-Spain 7, respectively, against the control group (IgG1, represented by bars with slanted lines, and IgG2a represented by white bars) throughout the experimental infection. The x-axis shows the days post-infection (PI).

### Example 5: Determination of the capability of conversion between the tachyzoite and bradyzoite stage of the isolate Nc-Spain 7 in in vitro studies

This section describes the assessment of the capability of *in vitro* conversion of tachyzoites to the bradyzoite stage of the isolate Nc-Spain 7 following the process described by Risco-Castillo et al., 2004, J Parasitol. 90(3):466-70.

Monolayers of cell line MARC-145 were cultivated on circular cover slips 10 mm in diameter, inside 24-well plates using as a medium DMEM culture medium supplemented with 10% bovine fetal serum, 15 mM HEPES (pH 7.2), 2 mM glutamine, 100 U/ml penicillin, 100 µg/ml streptomycin and 250 ng/ml amphotericin B. The cell monolayers were infected at 24 hours with tachyzoites of the isolate Nc-Spain 7 at a host:parasite infection dose of 2:1. Twenty-four hours after the infection, the medium was replaced with fresh medium containing 2% bovine fetal serum and 70 µM sodium nitroprussiate (Sigma) as a stressing agent inductor of the conversion into the bradyzoite stage. This medium was replaced daily for 7 days and the cultures were maintained at 37°-C and 5% CO₂.

Throughout the experiment the cell monolayers infected daily until day 3 and days 5 and 7 post-stress were fixed to perform a double-label indirect immunofluorescence assay using a rabbit polyclonal serum produced against recombinant protein TgBAG1 (αBAG1; 1:100 dilution), which reacts against the specific intracytoplasmatic antigen of bradyzoite called NcBAG1, and a monoclonal antibody developed in mice against the specific surface protein of tachyzoite called SAG1 (aSAG1, 1:150 dilution). Rabbit anti-IgG conjugated with fluorescein isothiocyanate (FICT) and mouse anti-IgG conjugated with tetramethyl rhodamine (TRIC) (Sigma), respectively, were used as secondary antibodies. The nuclei of the host cells and of the parasite were marked with DAPI.

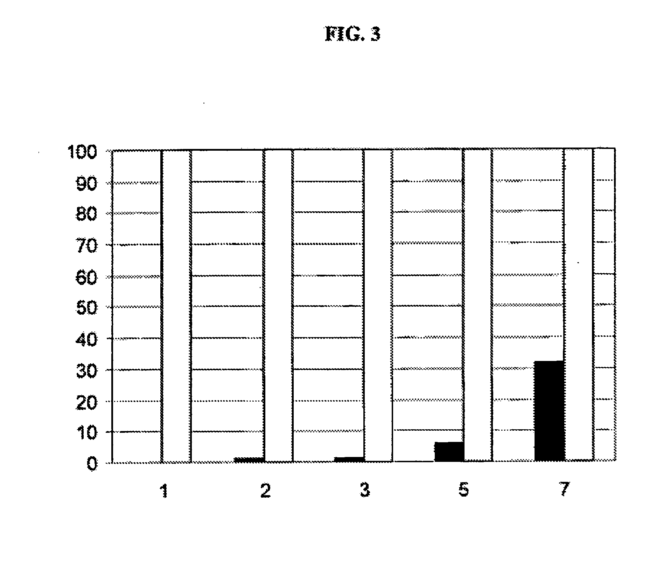

The crystals were observed in an inverted fluorescence microscope (Nikon Eclipse TE200) using a 100X immersion lens. The conversion was evaluated in duplicate for each day studied, randomly counting 10 fields in each crystal, to determine the expression of each specific protein. The expression of BAG1 was detected from the second day post-stress and progressively increased until observing 32% of BAG1-positive parasitophorous vacuoles on day 7 post-stress (Figure 3).

The degree of conversion between stages was established as the relative percentage of the number of parasitophorous vacuoles which expressed BAG1 versus the total parasitophorous vacuoles counted.

In Figure 3, the mean percentage of BAG1-positive vacuoles (black bars) and SAG1-positive vacuoles (white bars) is represented on the y-axis. The days of treatment with 70 µM of sodium nitroprussiate (SNP) are shown on the x-axis.

### Example 6: Immunohistochemical analysis of the capability of in vivo tachyzoite-bradyzoite conversion of the isolate Nc-Spain 7

This example describes the immunohistochemical analysis performed on the samples of the brain of the congenitally infected calf from which the isolate of the invention with which the capability of *in vivo* conversion between the stages of tachyzoite-bradyzoite of the isolate Nc-Spain 7 was obtained.

To that end, the different samples of nervous tissue maintained in 10% formalin solution were dehydrated and embedded in paraffin following the usual method. Consecutive multiple sections 3 to 6 µm thick of the paraffined samples were deparaffinized with xylene, rehydrated and subjected to treatment in 0.3% H₂O₂ in methanol for 30 minutes, for the purpose of inhibiting the endogenous peroxidase activity. They were subsequently treated calcium chloride-trypsin solution (pH 7.8) for 40 minutes at 37°C and blocked with goat serum for 30 minutes. The different sections were incubated with rabbit polyclonal serum developed against antigen BAG1, expressed in bradyzoite stage (αBAG1) at a 1:200 dilution for 12-18 hours at room temperature, or an anti-tachyzoites of N. *caninum* (αtachyzoite) rabbit polyclonal serum at a 1:3000 dilution. The samples were then immediately incubated with biotinylated anti-rabbit immunoglobulin IgG antibody for 30 minutes and then with the ABC-P solution for 40 minutes. It was developed by means of the use of diaminobenzidine as a substrate according to the manufacturer's instructions specified in the ABC kit (Vectastain ABC kit, Vector Lab.). Finally, the different sections were stained with hematoxylin, dehydrated and mounted for their microscopic observation.

The presence of bradyzoites located in cysts was detected in most of the analyzed sections. These bradyzoites were specifically recognized by the αBAG1 antibody.

### Example 7: Evaluation of the protective capability of an inactivated vaccine prepared with the isolate Nc-Spain 7

This example describes the development of an inactivated vaccine prepared with the isolate of the invention Nc-Spain 7 and its safety and efficacy were evaluated using an experimental murine model.

The antigen for the immunizations was prepared from tachyzoites obtained, collected and purified as described in Example 2, which were inactivated by means of their treatment with the binary ethyleneimine agent (BEI) at a concentration of 0.01 M in distilled water for a period of 96 hours at 4°C and, it was subsequently neutralized with a sodium thiosulfate solution. The vaccine product was then formulated by means of its adjuvanting with adjuvant A (Laboratorios Hipra S.A., Girona, Spain) at a concentration of antigen equivalent to 5x10⁵ inactivated tachyzoites per dose of inoculation in the Laboratorios Hipra S.A. (Gerona, Spain). Female mice of the BALB/c strain 8 weeks of age (Harlan Iberica, Barcelona) were used for the vaccination test.

Table IV comprises the different groups included in the vaccine test including a group vaccinated with the adjuvanted antigen (G1) or with the adjuvant without antigen (G2) which were challenged with a dose of experimental infection of 2x10⁶ tachyzoites of the isolate Nc-1, and two groups of control mice, one group not vaccinated and challenged (G3) and the other group not vaccinated or challenged (G4), which formed "not vaccinated" group during the inductor period. With respect to the vaccination regime, it consisted of two vaccine doses with 21 days of interval considering the first inoculation as day 0 of the experiment, and at 53 days post-inoculation the challenge was performed with 2x10⁶ tachyzoites of the isolate Nc-1, by subcutaneous route.

The safety of the vaccine product was evaluated by observing the animals daily, verifying if they produced local reactions in the inoculation area or systemic reactions due to the vaccination.

The efficacy of the vaccine was assessed by means of the evaluation of the humoral immune response induced in the vaccinated animals, morbidity and mortality, as well as the presence of the parasite in the central nervous system by the PCR technique.

**Table IV. Distribution of the groups of the vaccine test**

| **GROUP** | No. of animals | Adjuvant | Antigen dose (µg/mouse) (Nc-Spain 7) | Vaccination and revaccination | Experimental Infection (Nc-1) |
|---|---|---|---|---|---|
| **G1** | 16 | A | 5x10⁵ inactivated tachyzoites | + | 2x10⁶ |
| **G2** | 13 | A | 0 | + | 2x10⁶ |
| **G3** | 5 | - | 0 | - | 2x10⁶ |
| **G4** | 14 | - | 0 | - | - |

To evaluate the humoral immune response (IgG1 and IgG2a) developed in the different assayed groups, an indirect ELISA with soluble proteins of *N*. *caninum* was used as an antigen as described in Example 4. The immune response was studied for the inductor phase (vaccination process), sacrificing 5 animals in group G1, G2 and "not vaccinated" on day 23 post-inoculation. The mice of the different groups were sacrificed on day 30 post-challenge (day 83 post-inoculation). A blood sample was collected for the evaluation of the humoral immune response developed during the effector phase (challenge or infection phase with the parasite).

The presence of the parasite in the brain of the animals sacrificed on day 30 post-challenge was determined by means of the PCR technique. To that end, the extraction of the genomic DNA from 10-20 mg of nervous tissue of the different brains was performed by means of the Realpure Genomic DNA Extraction commercial test (Realpure, Durviz), following the manufacturer's instructions, and then the detection of the parasite was performed by means of the nested PCR technique of the ITS-1 region following the process described in Example 1.

With respect to the statistical analysis of the obtained data, the assessment of the differences between percentages was performed applying the Chi-square or Fisher F test by means of 2x2 contingency tables. In the case of the optical density values for IgG1 and IgG2a, the differences between the groups were analyzed by the one-factor Anova parametric test. When statistically significant differences were observed, the groups were compared two by two by means of Duncan's parametric test. Additionally and using Dunnett's parametric test, all the groups were compared with group G3 (not vaccinated and infected).

With respect to safety, a local reaction due to the vaccination was observed only in those groups inoculated with adjuvant A. However, adverse systemic reactions as a result thereof were not observed in any of the groups.

With respect to efficacy, (inactivity, bristling, rounded back, weakness and nervous symptomatology) were observed after the challenge clinical signs compatible with the infection by *N. caninum* in some animals (one mouse in group G1 and another mouse in group G3). Significant differences were not observed in any of the analyzed groups.

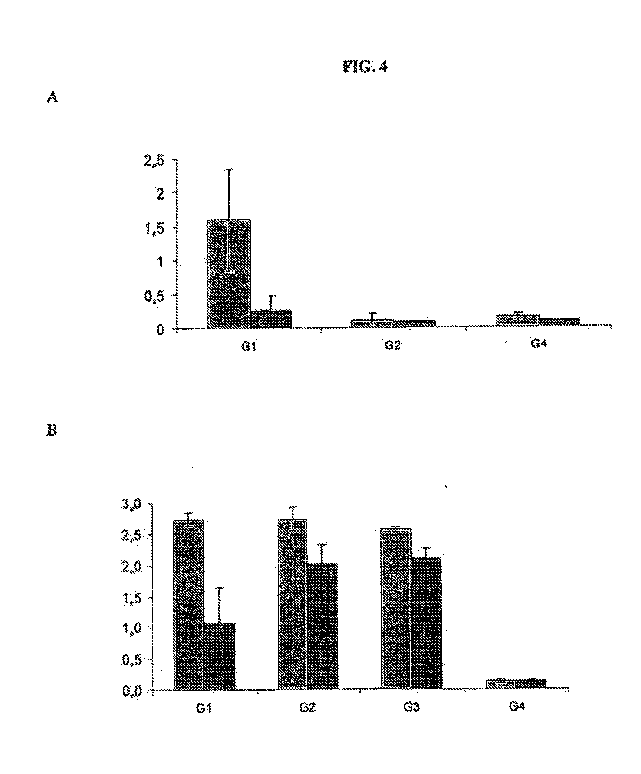

The highest levels of the isotypes IgG1 and IgG2a were detected in the inductor phase in the vaccinated group (G1). With respect to IgG1 significant differences were observed when comparing group G1 to G2 (one-factor Anova) and the "not vaccinated" group (G4) (Dunnett's test). In the case of the isotype IgG2a, no statistically significant differences were observed (Figure 4, Panel A). Elevated levels were detected during the effector phase for both isotypes IgG2a and IgG1 in all the challenged groups (G1, G2 and G3). With respect to IgG1, no statistically significant differences were observed when comparing the challenged groups, although with respect to the isotype IgG2a the detected levels in group G2 were significantly greater compared to group G1 (one-factor Anova) (Figure 4, Panel B).

In Figure 4, the optical density (OD), determined at 450 nm using the ELISA technique, is represented on the y-axis as the measurement of the antibodies IgG1 (grey bars) and IgG2a (black bars) specific for *N. caninum* produced by the mice of the different groups of the vaccine test for the inductor phase on day 23 post-inoculation (Panel A) and effector phase on day 30 after the challenge (Panel B). The different groups included in the vaccine test are shown on the x-axis.

The results obtained during the inductor period demonstrated the capability of the vaccine preparation of inducing an immunological response, at least a humoral type response, specific for *N. caninum.*

The presence of DNA of *N. caninum* was detected in a variable number of animals depending on the analyzed group (Table V). Statistically significant differences were not obtained when group G1 was compared to G2 or G2 was compared to G3 (P>0.05, Fisher F), although the result was statistically significant when group G1 (vaccinated and challenged) was compared to group G3 (not vaccinated and challenged) (P<0.05, Fisher F). Therefore, in the animals vaccinated with the isolate Nc-Spain 7 a significant decrease of the detectability of the parasite in the SNC was observed with respect to the control group not vaccinated and challenged, which implies that the immune response developed by the vaccine preparation is able to limit the multiplication of the parasite in this target organ.

**Table V. Results of nested PCR in the brain.**

| **GROUP** | **G1** | **G2** | **G3** | **G4** |
|---|---|---|---|---|
| POSITIVE ANIMALS/TOTAL | 4/11 | 6/8 | 5/5 | 0/9 |
| PERCENTAGE | 36.6% | 75% | 100% | 0% |

### Example 8: Application of the isolate Nc-Spain 7 in diagnostic serological tests

The use of the isolate of the invention Nc-Spain 7 in diagnostic techniques based on the detection of serological antibodies specific for *N. caninum,* and in this specific example, by means of its use in the immunoblot technique, is described.

Different samples of 10⁸ tachyzoites of the isolates Nc-Spain 7 and Nc-1, used in this study as the reference isolate, maintained at -80°C, obtained, collected and purified as described in Example 2, were resuspended in protein lysis buffer (2% of sulfate dodecyl sodium (SDS), 10% glycerol, 60 mM Tris-HCl (pH 6.8), 100 mM dithiothreitol (DTT) and 0.048% bromophenol blue), boiled for 10 minutes and subjected to electrophoresis in acrylamide gels. The antigen equivalent to 2x10⁷ tachyzoites per gel was thus resolved through a 4% bis/acrylamide packing gel (pH 6.8) then followed by a 12.5% acrylamide-DATD separating gel using the Mini-PROTEAN II System (Bio-Rad, California, USA). For the purpose of estimating the migration pattern of the different antigens subsequently detected, the Precision Plus Protein Standards Kaleidoscope marker (Bio-Rad) was also included. Then the proteins were transferred to a 0.22 µm nitrocellulose membrane using the Mini Trans-Blot Cell system (Bio-Rad).

After the treatment of the membranes in TBS blocking solution TBS (3% BSA in 0.05% TBS-Tween) were incubated with the plasmas obtained from experimentally infected mice and the sera of bovines naturally or experimentally infected, at a 1:50 and 1:20 dilution, respectively. A polyclonal antibody developed in rabbit for mouse IgG whole molecule at a 1:500 dilution (Sigma) and a monoclonal antibody for bovine IgG1 and IgG2 at a 1:400 dilution (Hipra S.A., Gerona, Spain), both conjugated with peroxidase, was used as a second antibody. After incubation with this second antibody, the antigen-antibody complexes formed were detected using the chromogenic 4-chloro-1-naphthol compound (Bio-Rad) as a substrate.

The mouse plasma samples used were obtained by means of the mixture at equal parts of the plasmas collected after day 32 post-infection of experimentally infected mice with 10⁶ tachyzoites of the isolates Nc-1, Nc-Liverpool and Nc-Spain 7 and 10⁷ tachyzoites of Nc-Spain 7, as described in Example 3. Likewise, the mixture of the plasmas of the control mice inoculated with PBS on day 32 post-infection was included as a control.

With respect to the bovine samples studied, sera of naturally infected animals, both precolostral sera obtained from congenitally infected calves, including the serum of the calf from which the isolate Nc-Spain 7 was obtained, and sera obtained from naturally infected cows, were included. Furthermore, sera obtained by means of the mixture at equal parts of the sera collected from experimentally infected heifers on day 70 of gestation with 10⁸ tachyzoites of the isolate Nc-1 were tested. The respective negative control sera were also included.

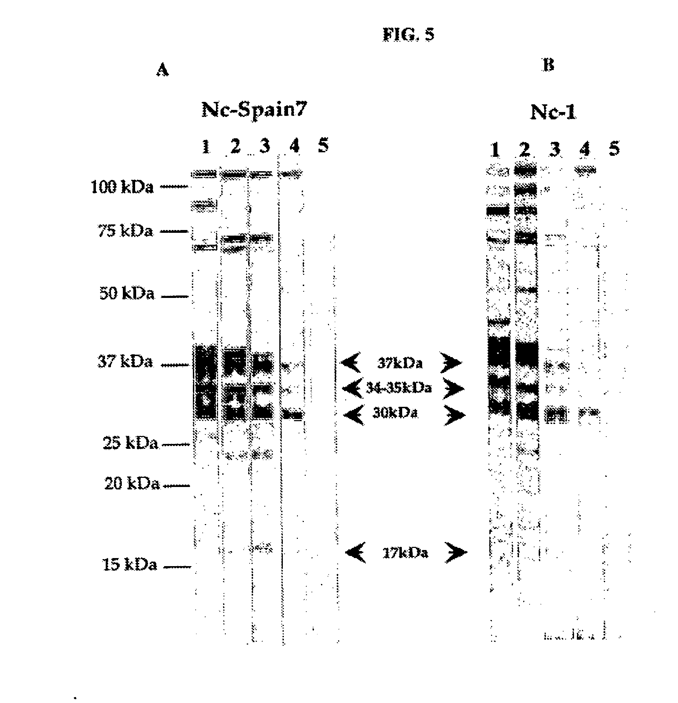

The antigenic profiles detected when using the plasma of the analyzed mice were identical in the extracts of both isolates, Nc-Spain 7 and Nc-1 (Figure 5). The main antigenic proteins detected in both isolates were 17, 24, 30, 34-35, 37, 39, 72-74 kDa, and some with an elevated molecular weight (> 80 kDa). Although the reaction was more intense and a larger number of bands was observed with the plasma of the mice infected with 10⁷ tachyzoites of the isolate Nc-Spain 7 compared to plasma of those infected with 10⁶, most of the immunodominant antigens of 30, 34-35 and 37 kDa described in previous studies (Atkinson et al., 1999, Parasitology 118 (Pt 4):363-370) were clearly detected.

Figure 5 shows the immunoblot analysis with the whole protein extract of the isolates of *N. caninum* Nc-Spain 7 (Panel A) and Nc-1 (Panel B) under reducing conditions with a mixture at equal parts of the plasmas obtained from experimentally infected mice as described next: Balb/c mice infected with 10⁶ tachyzoites of Nc-1 (line 1), Balb/c mice infected with 10⁶ tachyzoites of Nc-Liverpool (line 2), Balb/c mice infected with 10⁷ tachyzoites of Nc-Spain 7 (line 3), Balb/c mice infected with 10⁶ tachyzoites of Nc-Spain 7 (line 4) and Balb/c mice inoculated with PBS (line 5). The migration profile of the proteins corresponding to the Precision Plus Protein Standards Kaleidoscope marker (Bio-Rad) is indicated by means of lines with their corresponding molecular weight in kDa. The main immunodominant antigens detected are indicated by means of arrow tips, indicating the apparent molecular weight in kDa.

Antigenic profiles similar for both isolates were recognized also in the analysis performed with the bovine sera. The immunodominant antigens of 17, 28, 30, 34-35 and 37 kDa, identified in previous studies (Álvarez-García et al., 2002, Vet Parasitol. 107(1-2): 15-27) were detected with the naturally infected bovine sera. The antigenic profiles developed with the sera obtained from the infected experimentally heifers, in contrast, showed a different profile in which the reaction generated by a protein of 39 kDa stood out, but they were identical for both isolates.

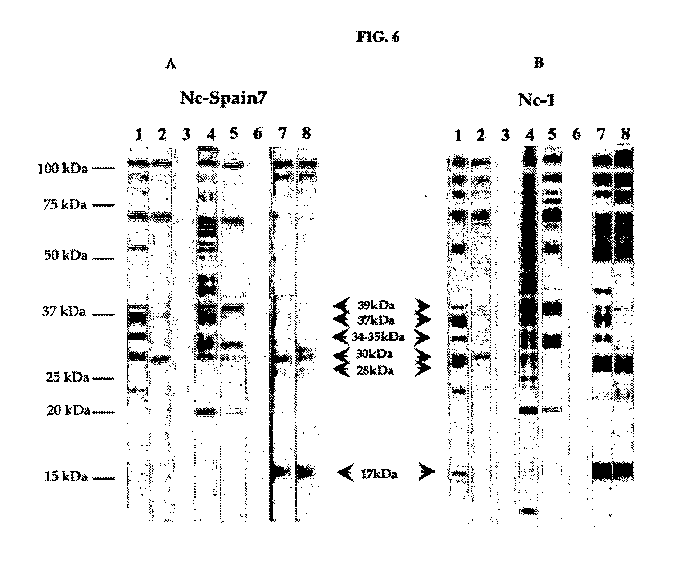

Figure 6 shows the immunoblot analysis with the whole protein extract of the isolates of *N. caninum* Nc-Spain 7 (Panel A) and Nc-1 (Panel B) under reducing conditions of the sera obtained from bovines, as described next: precolostral calf serum (1:1600 IFA titer) (line 1), precolostral calf serum (1:800 IFA titer) (calf of origin for the isolation of Nc-Spain 7) (line 2), negative control precolostral calf serum (line 3), mixture at equal parts of the sera of heifers experimentally infected with 10⁸ tachyzoites of Nc-1 (line 4 and 5), mixture at equal parts of the sera of heifers inoculated with PBS (line 6) and serum of naturally infected cows (line 7 and 8). The migration profile of the proteins corresponding to the Precision Plus Protein Standards Kaleidoscope marker (Bio-Rad) is indicated by means of lines with their corresponding molecular weight in kDa. The main immunodominant antigens detected are indicated by means of arrow tips, indicated the apparent molecular weight in kDa.

The results shown in this test confirm the usefulness of the isolate Nc-Spain 7 as an antigen in immunoassay tests for the detection of antibodies specific for *Neospora* in a biological sample. The series of sera used in the immunoblot test developed in the present example, formed by sera from animals naturally and experimentally infected with the *N. caninum* parasite, showed an identical antigenic pattern of detection in the cell extracts obtained from the reference isolate, Nc-1, and from Nc-Spain 7, including the detection of the set of antigenically predominant proteins or immunodominant antigens described in previous studies by other authors, which confirms their diagnostic usefulness.

## Claims

1. Parasite isolated from bovine nervous tissue, of the species *Neospora caninum,* called Nc-Spain 7, deposited in the Culture Collection of Algae and Protozoa with access number CCAP 2051/1.

2. Parasite isolated from bovine nervous tissue as defined in claim 1 for use in the diagnosis of the infection caused by *Neospora.*

3. The isolate for use according to claim 2, wherein the diagnosis is performed by means of immunological techniques.

4. The isolate for use according to claim 3, wherein the immunological technique used is an immunoenzymatic technique, an immunofluorescence technique, an immunoblot technique or an agglutination technique.

5. Parasite isolated from bovine nervous tissue as defined in claim 1 for use in the preparation of a vaccine for the treatment and/or prevention of the infection caused by *Neospora* in animals.

6. The isolate for use according to claim 5, wherein the manufacture of the vaccine is performed from the isolate Nc-Spain 7 in any of the stages of the biological cycle.

7. The isolate for use according to claim 5, wherein the manufacture of the vaccine is performed from the attenuated live, inactivated and/or fixed isolate Nc-Spain 7 in any of the stages of the biological cycle.

8. The isolate for use according to claim 5, wherein the manufacture of the vaccine is performed from a cell extract and/or lysate of the isolate Nc-Spain 7 in any of the stages of the biological cycle.

9. The isolate for use according to any one of claims 5 to 8, wherein the vaccine is prepared as an injectable in liquid formulations or in solid form for its subsequent reconstitution or as an oral formulation.

10. The isolate for use according to claim 9, wherein the vaccine comprises an adjuvant and/or one or several cytokines and/or immunostimulators.

11. Biologically pure cell cultures infected by the isolate Nc-Spain 7 of claim 1.

12. Biologically pure cell cultures according to claim 11, wherein the cultures are of vervet monkey kidney cells.

## Patentansprüche

1. Parasit, isoliert aus Rinder-Nervengewebe, der Spezies *Neospora caninum,* namens Nc-Spain 7, hinterlegt in den Kultursammlungen von Algen und Protozoen mit der Zugangsnummer CCAP 2051/1.

2. Parasit, isoliert aus Rinder-Nervengewebe, wie in Anspruch 1 definiert, zur Verwendung für die Diagnose der durch *Neospora* verursachten Infektion.

3. Isolat zur Verwendung nach Anspruch 2, wobei die Diagnose mittels immunologischer Techniken durchgeführt wird.

4. Isolat zur Verwendung nach Anspruch 3, wobei die immunologische Technik, die verwendet wird, eine Immun-Enzym Technik, eine Immunfluoreszenz-Technik, Immunoblot-Technik oder eine Agglutinations-Technik ist.

5. Parasit, isoliert aus Rinder-Nervengewebe, wie in Anspruch 1 definiert, zur Verwendung bei der Herstellung eines Impfstoffs zur Behandlung und/oder Verhinderung der Infektion, die durch *Neospora* beim Tier hervorgerufen wird.

6. Isolat zur Verwendung nach Anspruch 5, wobei die Herstellung des Impfstoffs von dem Isolat Nc-Spain 7 in einer der Phasen des biologischen Zyklus durchgeführt wird.

7. Isolat zur Verwendung nach Anspruch 5, wobei die Herstellung des Impfstoffs von dem abgeschwächten lebenden, inaktivierten und/oder fixierten Isolat Nc-Spain 7 in einer der Phasen des biologischen Zyklus durchgeführt wird.

8. Isolat zur Verwendung nach Anspruch 5, wobei die Herstellung des Impfstoffs von einem Zellextrakt und/oder Lysat des Isolats Nc-Spain 7 in einer der Phasen des biologischen Zyklus durchgeführt wird.

9. Isolat zur Verwendung nach einem der Ansprüche 5 bis 8, wobei der Impfstoff als eine Injizierbare in flüssigen Formulierungen oder in fester Form für die anschließende Wiederherstellung oder als orale Formulierung hergestellt wird.

10. Isolat zur Verwendung nach Anspruch 9, wobei der Impfstoff ein Adjuvans und/oder ein oder mehrere Zytokine und/oder Immunstimulanzien umfasst.

11. Biologisch reine Zellkulturen, die mit dem Isolat Nc-7 aus Anspruch 1 infiziert sind.

12. Biologisch reine Zellkulturen nach Anspruch 11, wobei die Kulturen aus Nierenzellen der Grünmeerkatze sind.

## Revendications

1. Parasite isolé à partir de tissu nerveux bovin, de l'espèce *Neospora caninum,* appelé Nc-Spain 7, déposé dans la collection « Culture Collection of Algae and Protozoa » avec le numéro d'accès CCAP 2051/1.

2. Parasite isolé à partir de tissu nerveux bovin tel que défini dans la revendication 1 pour une utilisation dans le diagnostic de l'infection provoquée par *Neospora.*

3. Isolat pour une utilisation selon la revendication 2, où le diagnostic est réalisé au moyen de techniques immunologiques.

4. Isolat pour une utilisation selon la revendication 3, où la technique immunologique utilisée est une technique immunoenzymatique, une technique d'immunofluorescence, une technique d'immunoblot ou une technique d'agglutination.

5. Parasite isolé à partir de tissu nerveux bovin tel que défini dans la revendication 1 pour une utilisation dans la préparation d'un vaccin pour le traitement et/ou la prévention de l'infection provoquée par *Neospora* chez des animaux.

6. Isolat pour une utilisation selon la revendication 5, où la fabrication du vaccin est réalisée à partir de l'isolat Nc-Spain 7 dans l'un quelconque des stades du cycle biologique.

7. Isolat pour une utilisation selon la revendication 5, où la fabrication du vaccin est réalisée à partir de l'isolat Nc-Spain 7 vivant atténué, inactivé et/ou fixé dans l'un quelconque des stades du cycle biologique.

8. Isolat pour une utilisation selon la revendication 5, où la fabrication du vaccin est réalisée à partir d'un extrait cellulaire et/ou d'un lysat de l'isolat Nc-Spain 7 dans l'un quelconque des stades du cycle biologique.

9. Isolat pour une utilisation selon l'une quelconque des revendications 5 à 8, où le vaccin est préparé sous la forme d'un injectable dans des formulations liquides ou sous forme solide pour sa reconstitution subséquente ou sous la forme d'une formulation orale.

10. Isolat pour une utilisation selon la revendication 9, où le vaccin comprend un adjuvant et/ou une ou plusieurs cytokines et/ou un ou plusieurs immunostimulants.

11. Cultures cellulaires biologiquement pures infectées par l'isolat Nc-Spain 7 selon la revendication 1.

12. Cultures cellulaires biologiquement pures selon la revendication 11, où les cultures sont des cellules de rein de singe vervet.
